# EUROPEAN PATENT APPLICATION

(11) **EP 3 569 617 A1**
(43) Date of publication of application: **20.11.2019**
(21) Application number: 18173145.6
(22) Date of filing: 18.05.2018
(51) Int. Cl.: C07K 16/28, A61K 39/395, A61P 37/04, A61P 3/08, A61P 29/00, A61P 17/00, A61P 25/28, A61K 39/00

(54) **PHARMACEUTICAL PREPARATION FOR USE IN TREATING EPSTEIN- BARR VIRUS POSITIVE PATIENTS WITH REACTIVATION PHENOMENON- ASSOCIATED DISEASES**

(71) Applicant: Trion Research GmbH, 82152 Martinsried (DE); Institut für Ernährung und Prävention GmbH, 81675 München (DE)
(72) Inventor: Lindhofer, Horst, 80639 München (DE); Jacob, Ursula, 81925 München (DE)
(74) Representative: Isarpatent

(57) **Abstract**

The present invention discloses an isolated trifunctional bispecific antibody for use in the treatment of a patient suffering from a reactivation of EBV in B cells, characterized in that the antibody has the following properties: (a) binding to a B cell via a B cell surface antigen; (b) binding to a T cell via a T cell surface antigen; (c) binding via its Fc-portion to an Fc receptor-positive cell. The present invention also discloses a pharmaceutical composition comprising the said isolated trifunctional bispecific antibody for use in the treatment of a patient suffering from a reactivation of EBV in B cells, and an ex-vivo method for preparing the said pharmaceutical composition. In the ex-vivo method, the autologous B cells of a patient suffering from a reactivation of EBV in B cells are incubated with said trifunctional bispecific antibody for a time-period sufficient to establish a physical interaction between said trifunctional bispecific antibody and B cells.

## Description

### Technical Field

The present invention relates to a medicament comprising an antibody for use in a method of treatment of a patient suffering from reactivation of Epstein-Barr virus (EBV) in B cells, to a pharmaceutical composition comprising the said antibody for said use, and also to an ex-vivo method for preparing the said pharmaceutical composition for said use.

### Background

Epstein-Barr virus (EBV) is a double-stranded DNA herpesvirus causing lifelong infection in a high proportion (>90%) of the world population. Primary infections usually occur during childhood and are mostly asymptomatic. In developed countries these infections are often delayed until adolescence in up to 50% of cases, which in some individuals produce severe symptoms persisting for years.

As many as half of these delayed primary infections are symptomatic, presenting as acute infectious mononucleosis (AIM) or glandular fever, manifested by fever, fatigue, malaise, pharyngitis and lymphadenopathy.

During the incubation period, the cycle of infection, lytic replication and reinfection initially proceeds without interference by cytotoxic CD8⁺ T cells, as it takes time to raise an adaptive immune response. As a result, the number of latently infected memory B cells during AIM can rise to half, or even higher, of the peripheral memory B-cell compartment (Hochberg et al., J.Virol., 78:5194, 2004).

It has been suggested that the difference between asymptomatic primary EBV infection and AIM is the higher number of EBV-infected B cells in AIM, with the symptoms being due to the massive destruction of EBV-infected B-cells by cytotoxic CD8⁺ T cells (Hadinoto et al., Blood, 111:1420, 2008).

EBV was the first human DNA virus to be recognized to have oncogenic potential. It has been found associated with various human malignancies, e.g., Burkitt's lymphoma (BL), undifferentiated nasopharyngeal carcinoma (NPC), salivary gland tumors or Hodgkin's disease (HD). But the list of EBV-associated diseases is even increasing.

Thus, EBV is increasingly discussed as a potential inducer of immune disorders and associated diseases after reactivation phenomenon.

The reason for such immune disorders may be localized in an uncontrolled repeated initiation of the lytic cycle (reactivation phenomenon) of EBV, which is in contrast to the normally stable latent status of EBV as found in healthy EBV-infected persons.

EBV infects almost exclusively B cells via the CD21 surface molecule. Therefore, we applied an *in situ* detection method of EBV mRNA inside infected cells to estimate the percentage of infected B cells to a given time point (taking blood sample). As in a healthy EBV-infected individual the frequency of infected B cells is in the range of 1-50/10e6 B cells (Cohen, NEJM, 2000), an e.g. thousand-fold higher frequency of infected B cells could give a strong hint for an acute reactivation phenomenon.

In 2003, Michael Pender hypothesized that infection of autoreactive B cells (up to 20% of B cells) by EBV could be the cause of inflammation in specific tissues, dependent on the specificity of the B-cell receptor of the infected autoreactive B cell (Pender, Trends Immunol., 24:584, 2003).

As a consequence, chronic inflammation could be induced in various tissues by EBV-infected and activated autoreactive B cells, especially in genetically susceptible elderly (in case of Alzheimer and Parkinson disease) as one possible mechanism, and could therefore be responsible for various diseases/symptoms with unsettled origin. Examples for such diseases/symptoms are e.g. Alzheimer disease (Licastro et al., Oncosience, 3: 135-142, 2016), Parkinson disease (Woulfe J., Neurol. Neuroimmunol. Neuroinflamm. 2016), chronic fatigue syndrome, repeated infections, sleeping disorders, night sweat, swollen lymphglands, chronic cystitis, chronic prostatitis, chronic inflammation of milk ducts, acne -like skin inflammation, hair loss, loss of concentration and memory problems.

In the last two decades, multiple publications presented also data for the involvement of EBV for a variety of autoimmune disorders as e.g. Rheumatoid Arthritis/ Sjögren's Syndrome; Multiple Sclerosis; Systemic Lupus Erythematosus; Diabetes Type 1; Crohn's disease/Chronic Colitis, Psoriasis, Vitiligo, Hashimoto's thyroiditis, Alopecia Areata/Generale and Myasthenia Gravis (Pender, Autoimmune Diseases, 2012).

### Rheumatoid Arthritis (RA)/Sjögren's Syndrome

One of the first direct evidences that EBV could be involved in the pathogenesis of RA was the investigation of Takei et al., from 1997. The authors detected EBV in 23.5% of specimens (n=34) of synovial lining cells by in situ hybridization targeting EBER-1. But in none of 20 osteoarthritis and 1 psoriatic arthritis used as controls (p<0.05).

A study by Blaschke et al., (2000) in general confirmed the results by Takei et al., as cells of synovial fluid harbored EBV-DNA in 30% of RA patients (n=55) compared to 16% of control cases (p=0.02). Additionally, this group found a two-fold increase of anti-EBNA-1 antibodies in comparison to healthy controls (p=0.029). Interestingly, 24% of RA patients had serological evidence for reactivated EBV infection in comparison to none of controls (p=0.028).

A further investigation by Balandraud et al., demonstrated in 2003 in a study with 84 RA patients and 69 normal controls using a real-time qPCR method, that EBV DNA load was increased almost 10-fold in RA patients compared to controls. The EBV load was stable over time and was not influenced by disease-modifying antirheumatic drugs or HLA-DR. Thus, in patients with RA, EBV, which is highly recognized by antibodies but never eliminated, is an ideal candidate to cause chronic immune complex disease and anti-EBV antibody responses should be considered as one of the chronic autoantibody responses that are most relevant to the development of RA (Van Boekel et al., Arthritis Res., 4:87, 2002). A recent review summarized the field (Füst, EuJMI, 4:267, 2011).

### Multiple Sclerosis

The involvement of EBV in the pathogenesis of MS is also intensely discussed. The following findings in MS patients are supporting such a hypothesis:
a) Accumulation of EBV-infected B cells and plasma cells in the brain (Serafini et al., J. Exp. Med, 2007),
b) Elevated anti-EBV antibody levels in serum and the cerebrospinal fluid (CSF) (Jaquiery et al., Eur. J. Immunol., 2010),
c) Alterations in EBV-specific CD8 T cell immunity (Jaquiery et al., 2010; Pender, J. Neurol. Neurosurg. Psychiatry, 2009) and
d) Increased amounts of EBV in saliva of MS patients compared to controls (Yea et al., Neurology, 2013).

Currently, the following main mechanisms are discussed to explain these findings and the possible role of EBV in the pathogenesis of MS:
(i) Cross reactivity (mimicry) between CNS and EBV antigens,
(ii) Impaired control of EBV infection with reactivation phenomenon
(iii) Bystander damage within the brain by EBV specific T cells and
(iv) EBV infection of autoreactive B cells, which are able to give costimulatory signals to autoreactive T cells in the brain.

### Type 1 + 2 Diabetes

The relationship between the onset of type 1 diabetes (T1D) and viral infection has been increasingly discussed in the last two decades. Viruses may be involved in the pathogenesis of T1D in several ways. In case of virus-induced autoimmunity EBV is discussed besides other viruses like e.g. mumps virus, Coxsackie virus, Rubella virus and Cytomegalovirus (Jun et al., Diabetes Metab. Res. Rev., 2003). Another reason for the development of diabetes mellitus (type 1 and 2) could be a chronic systemic inflammation. In this context, reactivation of HHV-6 and EBV is discussed. E.g. a recent investigation of Heaseker et al., (2013) found an association of high antibody titers of HHV-6 and EBV with diabetes mellitus.

Taken together, the existing data suggest that a weakened control of an existing EBV infection, together with genetic factors and further co-factors like e.g. low vitamin D levels, additional infections or by an unbalanced immune response against EBV due to a relative late infection (e.g. as a teenager or adult), can induce various autoimmune disorders.

Up to now, no therapeutic medicament against diseases associated with EBV reactivation is available, and there exists a need to provide a medicament for use in the treatment of a patient suffering from a reactivation of EBV in B cells.

Thus, it is a first object of the present invention is to provide a medicament for use in the treatment of a patient suffering from reactivation of EBV in B cells, particularly a medicament for use in the amelioration or treatment of diseases associated with reactivation of EBV in B cells. To achieve this object, the a trifunctional bispecific antibody is used which is characterized in that the antibody has the following properties: (a) binding to a B cell via a B cell surface antigen; (b) binding to a T cell via a T cell surface antigen; (c) binding via its Fc-portion to an Fc receptor-positive cell.

A second object of the present invention is to provide a pharmaceutical composition for use in the treatment of a patient suffering from a reactivation of EBV in B cells. To achieve this object, a pharmaceutical preparation is provided comprising said antibody and optionally pharmaceutically acceptable carriers and/or excipients for use in the treatment of a patient suffering from a reactivation of EBV in B cells.

A third object of the present invention is to provide an ex-vivo method for preparing the said pharmaceutical composition for use in the method of treatment of reactivation of EBV in B cells. To achieve this object, the said ex-vivo method comprises an incubation step comprising contacting autologous B cells of a patient suffering from a reactivation of EBV in B cells with said trifunctional bispecific antibody for a time-period sufficient to establish a physical interaction between said trifunctional bispecific antibody and B cells.

### Brief description of the invention

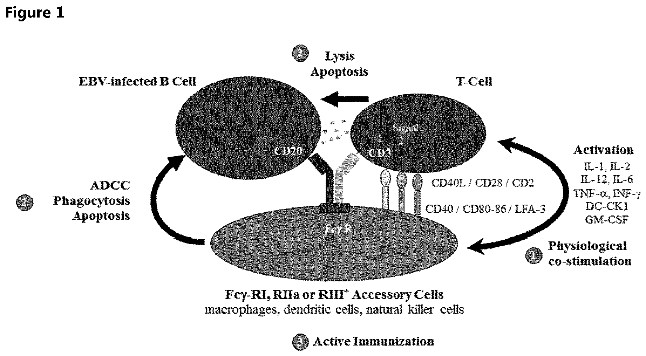

The inventors found that contacting *ex vivo* a trifunctional bispecific antibody with an enriched autologous B cell preparation containing B cells infected by EBV for a time period sufficient to establish a physical interaction between said trifunctional bispecific antibody and said EBV infected B cells, followed by a re-transfer of the treated autologous cells into the same patient's body, induces a retargeted killing of the EBV-infected B cells by T cells and accessory cells. The retargeted killing of an enriched autologous B cell population is shown in Example 1. As a consequence, the EBV-derived antigens will be phagocytosed by Fc -receptor positive professional antigen presenting cells (APC) like e.g. dendritic cells, Langerhans cells of the skin or macrophages, monocytes, natural killer cells and/or activated neutrophils. Eventually, these uptaken EBV-derived antigens will then be processed and newly presented by the professional APC leading to a polyclonal humoral and cellular immune response against the patient's EBV (Figure 1). As a result, the pre-existing but potentially weak or incomplete immune response against EBV will be enhanced and enables the patient to better control EBV by preventing or reducing its reactivation.

Further aspects and embodiments of the invention are disclosed in the dependent claims and can be taken from the following description and examples, without being limited thereto.

### Figures

The enclosed figures illustrate embodiments of the present invention and convey a further understanding thereof. In connection with the description they serve as explanation of concepts and principles of the invention. Other embodiments and further advantages can be derived from the figures.
Figure 1: Principle of the therapeutic vaccination involving a trifunctional bispecific antibody, which is able to bind to B cells, T cells and FcY receptor positive accessory cells, for use in the treatment of patients suffering from a reactivation of EBV in B cells and associated diseases and symptoms.
Figure 2: Trifunctional bispecific antibody mediated killing of enriched autologous B-cells *in vitro.*
Figure 3: *In situ* hybridization of enriched B-cells from the patient in Example 2 using EBV-EBER 1+2 probe.
Figure 4: *In situ* hybridization of enriched B-cells from the patient in Example 3 using EBV-EBER 1+2 probe.
Figure 5: *In situ* hybridization of enriched B-cells from the patient in Example 4 using EBV-EBER 1+2 probe.
Figure 6: *In situ* hybridization of enriched B-cells from the patient in Example 5 using EBV-EBER 1+2 probe.
Figure 7: *In situ* hybridization of enriched B-cells from the patient in Example 6 using EBV-EBER 1+2 probe.
Figure 8: *In situ* hybridization of enriched B-cells from the patient in Example 7 using EBV-EBER 1+2 probe.
Figure 9: *In situ* hybridization of enriched B-cells from the patient in Example 7 using EBV-EBER 1+2 probe.
Figure 10: *In situ* hybridization of enriched B-cells from the patient in Example 8 using EBV-EBER 1+2 probe.
Figure 11: *In situ* hybridization of enriched B-cells from the patient in Example 9 using EBV-EBER 1+2 probe.
Figure 12: *In situ* hybridization of enriched B-cells from the patient in Example 9 using EBV-EBER 1+2 probe.
Figure 13: *In situ* hybridization of enriched B-cells from the patient in Example 10 using EBV-EBER 1+2 probe.
Figure 14: *In situ* hybridization of enriched B-cells from the patient in Example 10 using EBV-EBER 1+2 probe.
Figure 15: *In situ* hybridization of enriched B-cells from the patient in Example 11 using EBV-EBER 1+2 probe.
Figure 16: *in situ* hybridization of enriched B-cells from the patient in Example 11 using EBV-EBER 1+2 probe.

### Detailed description of the invention

### Definitions

Although the present invention is described in detail below, it is to be understood that this invention is not limited to the particular methodologies, protocols and reagents described herein as these may vary. It is also to be understood that the terminology used herein is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" *e.g.,* a composition "comprising" X may consist exclusively of X or may include something additional *e.g*., X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" *e.g*., a composition which is "substantially free" from Y may be completely free from Y. Where necessary, the word "substantially" may be omitted from the definition of the invention.

All values given in the present disclosure are to be understood to be complemented by the word "about", unless it is clear to the contrary from the context.

In a first aspect of the present invention is provided an isolated trifunctional bispecific antibody for use in a method of treatment of a patient suffering from a reactivation of EBV in B cells, characterized in that the antibody has the following properties: (a) binding to a B cell via a B cell surface antigen; (b) binding to a T cell via a T cell surface antigen; (c) binding via its Fc-portion to an Fc receptor-positive cell.

In this regard, the trifunctional bispecific antibody for use according to the present invention may be generated in a mouse, rat, goat, sheep, rabbit, horse, donkey, pig, or other animals which can be immunized and are suitable for generating antibodies. Preferably, the antibody for use according to the present invention is generated in a mouse or rat.

The antibody for use according to the present invention may also be generated in transgenic animals. In this regard, the transgenic animal can be mammals, birds or fishes.

The antibody for use according to present invention may also be generated in cells which can secrete an antibody. Such cells include but are not limited to CHO, 293, COS or NS0 cells.

The term "antibody" encompasses various forms of antibodies, preferably monoclonal antibodies including, but not being limited to, whole antibodies, antibody fragments, human antibodies, chimeric antibodies, humanized antibodies and genetically engineered antibodies (variant or mutant antibodies) as long as the characteristic properties according to the invention are retained. Human or humanized monoclonal antibodies and recombinant antibodies, in particular recombinant monoclonal antibodies, are preferred. Thus, the antibody, according to the present invention is preferably a monoclonal antibody. Moreover, it is also preferred that the antibody is a multi-chain antibody, i.e. an antibody comprising more than one chain, which is thus different from a single-chain antibody.

The term "human antibody", as used herein, is intended to include antibodies having variable and constant regions derived from human immunoglobulin sequences. Human antibodies are well-known in the state of the art (van Dijk, M. A., and van de Winkel, J. G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon antigen challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 3340). Human antibodies can also be produced in phage display libraries (Hoogenboom, H. R., and Winter, G., J. Mol. Biol. 227 (1992) 381-388; Marks, J. D., et al., J. Mol. Biol. 222 (1991) 581-597). The techniques of Cole et al. and Boerner et al. are also available for the preparation of human monoclonal antibodies (Cole et al., Monoclonal Antibodies and Cancer Therapy, Alan R. Liss, p. 77 (1985); and Boerner, P., et al., J. Immunol. 147 (1991) 86-95). The term "human antibody" as used herein also comprises such antibodies which are modified, e.g. in the variable region, to generate the properties according to the invention.

As used herein, the term "recombinant antibody" is intended to include all antibodies, which do not occur in nature, in particular antibodies that are prepared, expressed, created or isolated by recombinant means, such as antibodies isolated from a host cell such as for example a CHO cell or from an animal (e.g. a mouse) or antibodies expressed using a recombinant expression vector transfected into a host cell. Such recombinant antibodies have variable and constant regions in a rearranged form as compared to naturally occurring antibodies.

As used herein, "trifunctional" antibodies are understood in the context of the present invention as a specific class of bispecific antibodies which recruit B cells and T cells, and simultaneously also recruit Fc receptor-positive cells. In the context of the present invention, the trifunctional bispecific antibody binds via its Fc-portion to Fc receptor-positive cells.

As used herein, the term "Fc-portion" refers to a sequence derived from the portion of an immunoglobulin heavy chain beginning in the hinge region just upstream of the papain cleavage site and ending at the C-terminus of the immunoglobulin heavy chain. Accordingly, an "Fc-portion" may be a complete Fc region or a part (e.g., a domain) thereof. Preferably, the "Fc moiety" mediates the full functionality of a complete Fc region, e.g. including Fc receptor binding. Thus, the antibody as used according to the present invention preferably comprises a complete Fc region, whereby a complete Fc region comprises at least a hinge domain, a CH2 domain, and a CH3 domain. The Fc-portion may also comprise one or more amino acid insertions, deletions, or substitutions relative to a naturally-occurring Fc region. For example, at least one of a hinge domain, CH2 domain or CH3 domain (or portion thereof) may be deleted. For example, an Fc-portion may comprise or consist of: (i) hinge domain (or portion thereof) fused to a CH2 domain (or portion thereof), (ii) a hinge domain (or portion thereof) fused to a CH3 domain (or portion thereof), (iii) a CH2 domain (or portion thereof) fused to a CH3 domain (or portion thereof), (iv) a hinge domain (or portion thereof), (v) a CH2 domain (or portion thereof), or (vi) a CH3 domain or portion thereof.

Preferably, the trifunctional bispecific antibody for use according to the present invention contains one or more binding sites in its Fc-portion for an Fc receptor.

More preferably, the trifunctional bispecific antibody contains one or more binding sites in its Fc-portion for Fcγ receptor type I, IIa and/or III.

Further preferably, the trifunctional bispecific antibody contains one binding site in its Fc-portion for Fcγ receptor type I, IIa and/or III.

Cells comprising Fcγ receptor type I, IIa and/or III include but are not limited to monocytes, macrophages, dendritic cells, natural killer cells and/or activated neutrophils. Preferably, the trifunctional bispecific antibody is capable of binding monocytes, macrophages, dendritic cells, natural killer cells and/or activated neutrophils by their Fcγ receptor type I, IIa and/or III.

The trifunctional bispecific antibody for use according to the invention may exhibit one of the following isotype combinations in its Fc-portion: rat-IgG2b/mouse-IgG2a, rat-IgG2b/mouse-IgG2b, rat-IgG2b/human-IgG1, or human-IgGl/human-IgGl-[hinge]-human IgG3*-[CH2-CH3], wherein * = caucasian allotypes G3m(b+g) = no binding to protein A.

Preferably, the trifunctional bispecific antibody is a rat/mouse bisipecific antibody and exhibits the isotype combination of rat-IgG2b/mosue-IgG2a in its Fc-portion.

As used herein, the term "bispecific" refers to the ability to bind to two different epitopes, i.e. on a T cell surface antigen and on a B cell antigen. Moreover, a single "specificity" may refer to one, two, three or more identical paratopes in a single antibody (the actual number of paratopes in one single antibody molecule is referred to as "valency"). For example, a single native IgG antibody is monospecific and bivalent, since it has two identical paratopes. As used herein, "paratope" refers to an epitope-binding site of the antibody. Thus, the term "bispecific antibodies" refers to antibodies having two different paratopes and the ability to bind to two different epitopes.

Preferably, the bispecific antibody according to the present invention may comprise four paratopes, wherein each two paratopes are identical (i.e. have the same specificity) and, thus, the antibody is bispecific and tetravalent (two identical paratopes for each of the two specificities). Thus, "two specificities" may be realized by two, three, four five, six, eight, ten, twelve or more paratopes as long as they refer to only two specificities. Most preferably a bispecific antibody comprises one single paratope for each specificity, i.e. the bispecific antibody comprises in total two paratopes. It is also preferred that the bispecific antibody comprises two identical paratopes for each of the two specificities, i.e. the bispecific antibody comprises in total four paratopes. Preferably the bispecific antibody comprises three (identical) paratopes for each of the two specificities, i.e. the bispecific antibody comprises in total six paratopes.

As used herein, the term "antigen" refers to any structural substance which serves as a target for the receptors of an adaptive immune response, in particular as a target for antibodies, T cell receptors, and/or B cell receptors. An "epitope", also known as "antigenic determinant", is the part (or fragment) of an antigen that is recognized by the immune system, in particular by antibodies, T cell receptors, and/or B cell receptors. Thus, one antigen has at least one epitope, i.e. a single antigen has one or more epitopes. An antigen may be (i) a peptide, a polypeptide, or a protein, (ii) a polysaccharide, (iii) a lipid, (iv) a lipoprotein or a lipopeptide, (v) a glycolipid, (vi) a nucleic acid, or (vii) a small molecule drug or a toxin. Thus, an antigen may be a peptide, a protein, a polysaccharide, a lipid, a combination thereof including lipoproteins and glycolipids, a nucleic acid (e.g. DNA, siRNA, shRNA, antisense oligonucleotides, decoy DNA, plasmid), or a small molecule drug (e.g. cyclosporine A, paclitaxel, doxorubicin, methotrexate, 5-aminolevulinic acid), or any combination thereof. Preferably, the antigen is selected from (i) a peptide, a polypeptide, or a protein, (ii) a polysaccharide, (iii) a lipid, (iv) a lipoprotein or a lipopeptide and (v) a glycolipid; more preferably, the antigen is a peptide, a polypeptide, or a protein.

As used herein, "a B cell surface antigen" refers to a B cell surface-associated antigen or a B cell surface-specific antigen, and a "T cell surface antigen" refers to a T cell surface-associated antigen or a T cell-specific antigen. In the context of the present invention, a B cell surface antigen or a T cell surface antigen may be a cluster of differentiation (CD) molecules. CD molecules are markers on the cell surface which are useful for identifying leukocytes. The bispecific antibody for use according to the present invention may bind to a B cell via a B cell surface antigen selected from the group consisting of CD19, CD20, CD21, CD22, CD23, CD24, CD37, CD38, CD72, CD75, CD78, CD79 and CD80. It means that the antibody for use according to the present invention preferably comprise a paratope which can recognize and bind to an epitope of a B cell surface antigen selected from the group consisting of CD19, CD20, CD21, CD22, CD23, CD24, CD37, CD38, CD72, CD75, CD78, CD79 and CD80. This specificity preferably promotes the recruitment of B cells.

Preferably, the B cell surface antigen is CD20. It means that the antibody for use according to the present invention further preferably comprises a paratope which can recognize and bind to an epitope of CD20.

The bispecific antibody for use according to the present invention may bind to a T cell via a T cell surface antigen selected from a group consisting of CD2, CD3, CD4, CD8, CD28, CD40L and CD44. It means that the antibody for use according to the present invention preferably comprises a paratope which can recognize and bind to an epitope of a T cell surface antigen selected from the group consisting of CD2, CD3, CD4, CD8, CD28, CD40L and CD44. This specificity preferably promotes the recruitment of T cells.

Preferably, the T cell surface antigen is CD3. It means that the antibody for use according to the present invention further preferably comprises a paratope which can recognize and bind to an epitope of CD3.

The bispecific antibody for use according to the present invention may bind: (1) by its first paratope to an epitope of the B cell surface antigen selected from the group consisting of CD19, CD20, CD21, CD22, CD23, CD24, CD37, CD38, CD72, CD75, CD78, CD79 and CD80, preferably CD20; (2) at the same time by its second paratope to an epitope of the T cell surface antigen selected from the group consisting of CD2, CD3, CD4, CD8, CD28, CD40L and CD44, preferably CD3.

The bispecific antibody for use according to the present invention may comprise one paratope against CD3 and one paratope against CD20 (anti-CD3 x anti-CD20). The said antibody may comprise one paratope against CD3 and one paratope against CD19 (anti-CD3 x anti-CD19). The said antibody may comprise one paratope against CD3 and one paratope against CD22 (anti-CD3 x anti-CD22). The said antibody may comprise one paratope against CD3 and one paratope against CD38 (anti-CD3 x anti-CD38). It means the antibody for use according to the present invention is preferably selected from a group consisting of anti-CD3 x anti-CD20, anti-CD3 x anti-CD19, anti-CD3 x anti-CD22, anti-CD3 x anti-CD38 bispecific antibodies.

More preferably, the bispecific antibody for use according to the present invention comprises is anti-CD3 x anti-CD20 bispecific antibody.

Accordingly, the trifunctional bispecific antibody for use according to the present invention may comprises: (1) one paratope which can recognize and bind to an epitope of a B cell surface antigen selected from the group consisting of CD19, CD20, CD21, CD22, CD23, CD24, CD37, CD38, CD72, CD75, CD78, CD79 and CD80, preferably CD20; (2) one paratope which can recognize and bind to an epitope of a T cell surface antigen selected from the group consisting of CD2, CD3, CD4, CD8, CD28, CD40L and CD44, preferably CD3; (3) one Fc-portion which can bind to an Fc receptor-positive cells, preferably one Fc-portion containing a binding site for Fcγ receptor type I, IIa and/or III.

Preferably, the trifunctional bispecific antibody for use according to the present invention comprises: (1) one paratope which can recognize and bind to an epitope of a B cell surface antigen selected from the group consisting of CD19, CD20, CD21, CD22, CD23, CD24, CD37, CD38, CD72, CD75, CD78, CD79 and CD80, preferably CD20; (2) one paratope which can recognize and bind to an epitope of a T cell surface antigen selected from the group consisting of CD2, CD3, CD4, CD8, CD28, CD40L and CD44, preferably CD3; (3) one Fc-portion which can bind to an Fc receptor-positive cells, preferably one Fc-portion containing a binding site for Fcγ receptor type I, IIa and/or III, and more preferably the Fc-portion contains one isotype combination selected from a group consisting of rat-IgG2b/mouse-IgG2a, rat-IgG2b/mouse-IgG2b, rat-IgG2b/human-IgG1, or human-IgGl/human-IgGl-[hinge]-human IgG3*-[CH2-CH3], wherein * = caucasian allotypes G3m(b+g) = no binding to protein A, even more preferably the Fc-portion contains the isotype combination of rat-IgG2b/mouse-IgG2a.

The trifunctional bispecific antibody for use according to the present invention may be selected from a group consisting of anti-CD3 x anti-CD20, anti-CD3 x anti-CD19, anti-CD3 x anti-CD22, anti-CD3 x anti-CD38 bispecific antibodies, preferably with the isotype combination selected from a group consisting of rat-IgG2b/mouse-IgG2a, rat-IgG2b/mouse-IgG2b, rat-IgG2b/human-IgG1, or human-IgGl/human-IgGl-[hinge]-human IgG3*-[CH2-CH3], wherein * = caucasian allotypes G3m(b+g) = no binding to protein A, even more preferably with the isotype combination of rat-IgG2b/mouse-IgG2a.

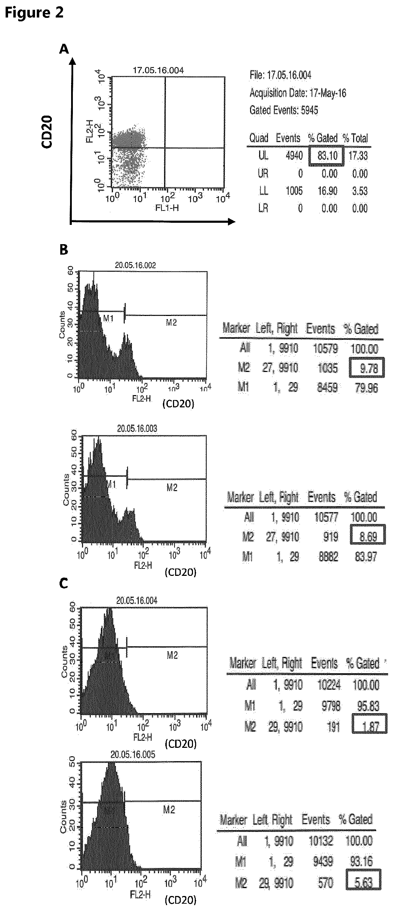

Preferably, a bispecific antibody in the context of the present invention may be of any bispecifc antibody format, e.g., as described in Spiess C., Zhai Q. and Carter P.J. (2015) Molecular Immunology 67: 95-106. For example, bispecific antibodiesmay be whole antibodies, such as whole IgG-like molecules, or fragments thereof which are not whole antibodies but retain antibody properties. These may be small recombinant formats, e.g. as tandem single chain variable fragment molecules, diabodies, single chain diabodies, bispecific T-cell engagers and various other derivatives of these (e.g. Byrne H. et al., 2013, Trends Biotech, 31 (11): 621-632 with Figure 2 showing various bispecific antibody formats). Several bispecific antibody formats can redirect effector cells against target cells that play key roles in disease processes. For example, several bispecific antibody formats can retarget effector cells towards B cells and a variety of bispecific antibody constructs were designed to retarget cells of the immune system, for example by binding to and triggering Fc receptors on the surface of effector cells or by binding to T cell receptor complexes.

The antibody for use according to the present invention may be of any antibody format. Examples of bispecific antibody formats include, but are not limited to, quadroma, chemically coupled Fab (fragment antigen binding), and BiTE® (bispecific T cell engager). In one embodiment of the present invention the antibody used is preferably a BiTE® (bispecific T cell engager).

Thus, the antibody for use according to the present invention may be selected from the group comprising Triomabs; hybrid hybridoma (quadroma); Multispecific anticalin platform (Pieris); Diabodies; Single chain diabodies; Tandem single chain Fv fragments; TandAbs, Trispecific Abs (Affimed) (105-110 kDa); Darts (dual affinity retargeting; Macrogenics); Bispecific Xmabs (Xencor); Bispecific T cell engagers (Bites; Amgen; 55kDa); Triplebodies; Tribody = Fab-scFv Fusion Protein (CreativeBiolabs) multifunctional recombinant antibody derivates (110 kDa); Duobody platform (Genmab); Dock and lock platform; Knob into hole (KIH) platform; Humanized bispecific IgG antibody (REGN1979) (Regeneron); Mab² bispecific antibodies (F-Star); DVD-Ig = dual variable domain immunoglobulin (Abbvie); kappa-lambda bodies; TBTI = tetravalent bispecific tandem Ig; and CrossMab.

The antibody for use according to the present invention may be selected from bispecific IgG-like antibodies comprising CrossMab; DAF (two-in-one); DAF (four-in-one); DutaMab; DT-IgG; Knobs-in-holes common LC; Knobs-in-holes assembly; Charge pair; Fab-arm exchange; SEEDbody; Triomab; LUZ-Y; Fcab; and Orthogonal Fab. These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

The antibody for use according to the present invention may be selected from IgG-appended antibodies with an additional antigen-binding moiety comprising DVD-IgG; IgG(H)-scFv; scFv-(H)IgG; IgG(L)-scFv; scFV-(L)IgG; IgG(L,H)-Fv; IgG(H)-V; V(H)-IgG; IgG(L)-V; V(L)-IgG; KIH IgG-scFab; 2scFv-IgG; IgG-2scFv; scFv4-Ig; scFv4-Ig; Zybody; and DVI-IgG (four-in-one). These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

The antibody for use according to the present invention may be selected from bispecific antibody fragments comprising Nanobody; Nanobody-HAS; BiTE; Diabody; DART; TandAb; scDiabody; sc-Diabody-CH3; Diabody-CH3; Triple Body; Miniantibody; Minibody; TriBi minibody; scFv-CH3 KIH; Fab-scFv; scFv-CH-CL-scFv; F(ab')2; F(ab')2-scFv2; scFv-KIH; Fab-scFv-Fc; Tetravalent HCAb; scDiabody-Fc; Diabody-Fc; Tandem scFv-Fc; and Intrabody. These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

The antibody for use according to the present invention may be selected from bispecific fusion proteins comprising Dock and Lock; ImmTAC; HSAbody; scDiabody-HAS; and Tandem scFv-Toxin. These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

The antibody for use according to the present invention may be selected from bispecific antibody conjugates comprising IgG-IgG; Cov-X-Body; and scFv1-PEG-scFv2. These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

It is also preferred, that the antibody for use according to the present invention is selected from the group consisting of a bispecific T-cell engager (BiTE™) and a bispecific trifunctional antibody.

It is also preferred that the antibody for use according to the invention comprises at least two different single-chain variable fragment (scFvs). An scFv is herein understood as a fusion protein of the variable regions of the heavy (VH) and light chains (VL) of immunoglobulins, connected with a short linker peptide. Said peptide typically comprises at least 5, preferably at least 10, more preferred about 25 amino acids. The linker is usually rich in glycine for flexibility, as well as serine or threonine for solubility, and can either connect the N-terminus of the VH with the C-terminus of the VL, or vice versa. A scFv may retain the specificity of the original immunoglobulin, despite removal of the constant regions and the introduction of the linker. Typically, a scFv can be created directly from subcloned heavy and light chains derived from a hybridoma. ScFvs are generally used, e.g., in flow cytometry, immunohistochemistry, and as antigen-binding domains of artificial T cell receptors. In the context of the present invention, they are preferably used as antigen-binding domains of artificial T cell receptors.

Preferably, the antibody for use according to the present invention has an IgG-like format (based on IgG, also referred to as "IgG type"), whereby an antibody having an IgG-like format usually comprises two heavy chains and two light chains. In general, Immunoglobulin G (IgG) is known as a type of antibody. It is understood herein as a protein complex composed of four peptide chains-two identical heavy chains and two identical light chains arranged in a Y-shape typical of antibody monomers. Each IgG has typically two antigen binding sites, which may be different or identical. Representing about 75% of serum antibodies in humans, IgG is the most common type of antibody found in the circulation. Physiologically, IgG molecules are created and released by plasma B cells.

Examples of an antibody having an IgG-like format include a quadroma and various IgG-scFv formats (cf: Byrne H. et al. (2013) Trends Biotech, 31 (11): 621-632; Figure 2A-E), whereby a quadroma is preferred, which is preferably generated by fusion of two different hybridomas. Within the IgG class, antibodies may preferably be based on the IgG1, IgG2, IgG3 or IgG4 subclass, whereby an antibody based on IgG1 (also referred to as "IgG1 type") is preferred. The multispecific antibodies or antigen binding fragments, such as bispecific antibodies, for use according to the present invention may alternatively be based on any immunoglobulin class (e.g., IgA, IgG, IgM etc.) and subclass (e.g. IgA1, IgA2, IgG1, IgG2, IgG3, IgG4 etc.)

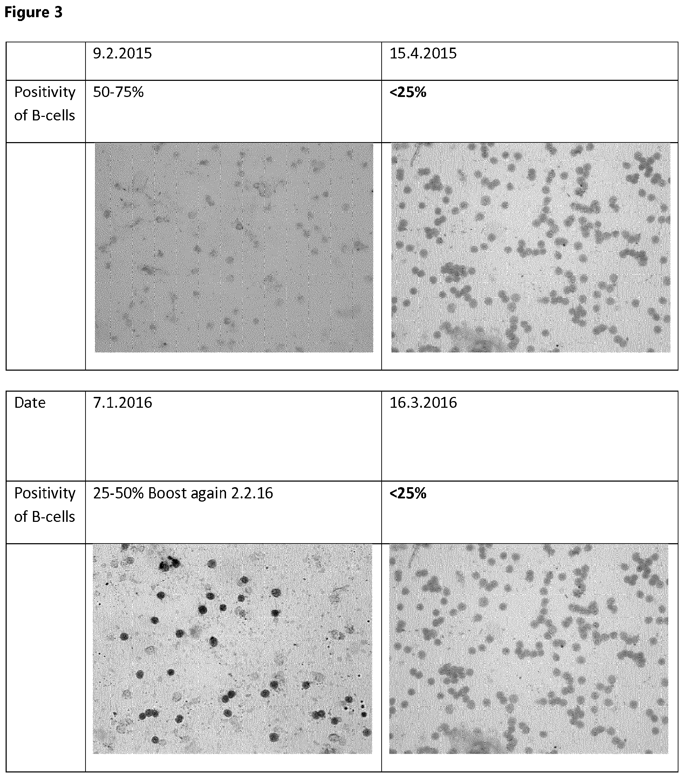

Preferred bispecific IgG-like antibody formats comprise for example hybrid hybridoma (quadroma), knobs-into-holes with common light chain, various IgG-scFv formats, various scFv-IgG formats, two-in-one IgG, dual V domain IgG, IgG-V, and V-IgG, which are shown for example in Figure 3c of Chan, A.C. and Carter, P.J. (2010) Nat Rev Immu 10: 301-316 and described in said article. Further preferred bispecific IgG-like antibody formats include for example DAF, CrossMab, IgG-dsscFv, DVD, IgG-dsFV, IgG-scFab, scFab-dsscFv and Fv2-Fc, which are shown in Fig. 1A of Weidle U.H. et al. (2013) Cancer Genomics and Proteomics 10: 1 - 18 and described in said article. Further preferred bispecific IgG-like antibody formats include DAF (two-in-one); DAF (four-in-one); DutaMab; DT-IgG; Knobs-in-holes assembly; Charge pair; Fab-arm exchange; SEEDbody; Triomab; LUZ-Y; Fcab; Orthogonal Fab; DVD-IgG; IgG(H)-scFv; scFv-(H)IgG; IgG(L)-scFv; scFV-(L)IgG; IgG(L,H)-Fv; IgG(H)-V; V(H)-IgG; IgG(L)-V; V(L)-IgG; KIH IgG-scFab; 2scFv-IgG; IgG-2scFv; scFv4-Ig; scFv4-Ig; Zybody; and DVI-IgG (four-in-one) as shown and described for example in Spiess C., Zhai Q. and Carter P.J. (2015) Molecular Immunology 67: 95-106, in particular Fig. 1 and corresponding description, e.g. p. 95 - 101.

The trifunctional bispecific antibody for use according to the present invention may be produced by three main methods: (i) chemical conjugation, which involves chemical crosslinking; (ii) fusion of two different hybridoma cell lines; or (iii) genetic approaches involving recombinant DNA technology. The fusion of two different hybridomas produces a hybrid-hybridoma (or "quadroma") secreting a heterogeneous antibody population including bispecific molecules.

Alternative approaches may include chemical conjugation of two different mAbs and/or smaller antibody fragments. Oxidative reassociation strategies to link two different antibodies or antibody fragments were found to be inefficient due to the presence of side reactions during reoxidation of the multiple native disulfide bonds. Current methods for chemical conjugation focus on the use of homo- or hetero-bifunctional crosslinking reagents. Recombinant DNA technology has yielded the greatest range of bispecific antibodies, through artificial manipulation of genes and represents the most diverse approach for bispecific antibody generation (45 formats in the past two decades; cf. Byrne H. et al. (2013) Trends Biotech, 31 (11): 621-632).

### Protocols of method of treatment

For the use of the trifunctional bispecific antibody in the context of the present invention, a patient with EBV infection may need to be identified. Due to the reactivation of EBV in B cells, the patients may suffer from a variety of disorders/diseases associated with said EBV-reactivation phenomenon, e.g. Chronic fatigue syndrome, migraine headaches, depression, sleeping disorders, insomnia, allergic rhinitis, chronic asthma, metabolic syndrome, lactose and gluten allergies, multiple allergies (mainly running nose, itching), lymphadenopathy, lymphoma, autoimmune disorders like e.g. Hashimoto, Sjögren-syndrome, psoriasis, irregular menstruation, ovarian cysts, chronical ductal inflammation, endometriosis, bladder dysfunction, secondary open angle glaucoma, chronic cystitis, chronic prostatitis.

In a first step, autologous B cells are isolated. For instance these autologous B cells may be obtained from autologous mononuclear cells of peripheral blood (PBMC) which are isolated from a patient, followed by an enrichment of autologous B cells from the isolated PBMC.

PBMC is any peripheral blood cell and comprises B cells, T cells, natural killer cells and monocytes. These cells can be isolated from whole blood by performing e.g. standard Ficoll density centrifugation. After the isolation of PBMC, the B cells contained in the PBMC may be enriched through using e.g. proper immunomagnetic beads. Subsequently, chromogenic in *situ* hybridization may be performed to detect the expression of EBV-specific RNA in the enriched B cells, to identify the infection of EBV in B cells. Preferably, the EBV-specific RNA is, but is not limited to, EBER, EBNA1 or EBNA2 RNA.

For the use of the trifunctional bispecific antibody in the context of the present invention, the antibody may be administered during incubation preferably in an amount of 0.1 - 100 µg, more preferably in an amount of 0.4-50 µg, further preferably in an amount of 0.6-20 µg, even further preferably in an amount of 0.8-10 µg. This is also the amount which is administered to the patient.

In the second aspect of the present invention, a pharmaceutical composition is disclosed for use in a method of treatment of patients suffering from a reactivation of EBV in B cells, wherein the composition comprises the trifunctional bispecific antibody as disclosed above.

Preferably, the pharmaceutical composition for use comprises also pharmaceutically acceptable carriers and/or excipients, including for example binders, lubricants, disintegrating agents, fillers and diluents.

The pharmaceutical composition for use comprises an autologous cell preparation of enriched B cells of a patient suffering from a reactivation of EBV in B cells. The enriched B cells can be prepared by any of the methods stated above.

In a further embodiment of the invention, the said pharmaceutical composition for use according to the present invention additionally comprises an autologous cell preparation of PBMC of the same patient. The PBMC can be isolated from whole blood as described above, for instance by performing standard Ficoll density centrifugation.

In a still further embodiment, the said pharmaceutical composition for use according to the present invention is in the form of a kit-of-parts, preferably comprising the trifunctional bispecific antibody as disclosed above, preparation of enriched autologous B cells from a EBV infected patient, and preparation of autologous PBMC from the same patient, wherein at least two of the components are provided in separate containers. Preferably, the said B cells are incubated with said trifunctional bispecific antibody for a time-period sufficient to establish a physical interaction between said trifunctional bispecific antibody and B cells.

According to the third aspect of the present invention, an *ex-vivo* method is disclosed for preparing the said pharmaceutical composition for use according to the present invention, characterized in that the method comprises an incubation step comprising contacting autologous B cells, from an autologous cell preparation of enriched B cells, of a patient suffering from a reactivation of EBV in B cells with the trifunctional bispecific antibody disclosed above for a time-period sufficient to establish a physical interaction between said trifunctional bispecific antibody and B cells. Preferably, the B cells are prepared by any of the methods stated above. The time-period for incubation of the said autologous B cells with the said trifunctional bispecific antibody may be between 1 min to 60 min, preferably between 1min to 20 min, more preferably between 5 min to 20 min, even more preferably between 8 min to 15 min, further preferably between 10 min to 15 min, e.g. 10, 11, 12, 13, 14 or 15 minutes.

Optionally, the said *ex-vivo* method further comprises adding PBMC of the same patient with reactivation of EBV in B cells to the incubated autologous B cells, for a time-period sufficient to establish a physical interaction between said trifunctional bispecific antibody and PBMC. The PBMC and the autologous B cells may be mixed and incubated with and the antibody either simultaneously or consecutively. The PBMC can be isolated from whole blood by performing e.g. standard Ficoll density centrifugation. The time-period for incubation of the said PBMC with the said incubated autologous B cells and trifunctional bispecific antibody may be between 1 min to 60 min, preferably between 1min to 20 min, more preferably 5 min to 20 min, even more preferably between 8 min to 15 min, further preferably between 10 min to 15 min, e.g. 10, 11, 12, 13, 14 or 15 minutes. In the present invention, the trifunctional bispecific antibodies are able to recruit and activate (i) T cells and (ii) Fc-receptor expressing cells, such as accessory immune cells, for example monocytes, macrophages, natural killer cells, dendritic cells, and/or activated neutrophils, and other Fc receptor expressing cells, simultaneously at the (iii) targeted B cells. The simultaneous activation of these different classes of effector cells results in efficient killing of the EBV infected B cells by various mechanisms such as, for example, phagocytosis and perforin-mediated cytotoxicity. Typically, the net effect of the trifunctional antibody, which comprises an Fc receptor, is linking T cells and Fc receptor positive cells to target B cells, i.e. EBV infected B cells, leading to the destruction of the virus infected cells.

Trifunctional antibodies evoke the removal of targeted cells in particular by means of (i) antibody-dependent cell-mediated cytotoxicity, (ii) T-cell mediated cell killing, and (iii) induction of anti-viral immunity. However, only the first mode of action is actually executed by conventional (monoclonal and monospecific) antibodies. In contrast to conventional antibodies, the trifunctional bispecific antibodies in the present invention have a higher cytotoxic potential and they even bind to antigens, which are expressed relatively weakly. Thus, the trifunctional bispecific antibodies in the present invention are at an equivalent dose more potent (more than 1,000-fold) in eliminating targeted cells compared to conventional antibodies.

### Material and Methods:

### PBMC preparation and B-cell enrichment

Mononuclear cells of peripheral blood (PBMC) were isolated from non-coagulated EDTA or Heparin-blood by standard Ficoll density centrifugation applying human Pancoll solution (Pan Biotech, Germany). Isolated PBMC were washed two times with phosphate buffered saline (PBS) (Pan Biotech. Germany). Erythrocytes were lysed with erythrocytes lysis buffer. Approximately, 1 x 10Exp6 PBMC could be isolated from 1 ml blood. B-cells of PBMC preparations were enriched by immunomagnetic beads using CELLection™ Pan Mouse IgG kit (Dynal Biotech, Germany): First, B-cells of PBMC preparations (1 x10Exp7/ml) were labeled with an anti-CD20 monoclonal mouse antibody (TPA10, Trion Research). Then, labeled B-cells were isolated by addition of magnetic beads coupled with anti- mouse IgG. Since the catching antibody was linked to the beads via a DNA-linker, captured B-cells could be released from the beads by enzymatic cleavage with DNAse. Finally, cells were washed several times with PBS until no more residual beads were visible. Efficacy of B-cell enrichment was controlled by FACS-analysis and usually ranged between 70 - 99%.

### Chromogenic in situ hybridization (CISH) for the detection of EBV-specific EBER RNA

Latently EBV-infected B-cells and PBMC that express untranslated EBV-specific EBER1 and EBER 2 RNA were detected by CISH applying a digoxygenin-labeled EBER RNA-specific oligonucleotide probe (Zytovision, ZytoFast EBV probe, IVD medical device, Germany). PBMC and B-cells were prepared as described above. In situ hybridization was performed according to the manufacturer's instructions. Cytospins were analyzed by microscopy. Positively stained cells appeared red

### Detection of gp350 antigen on B-cells

250 x 10Exp3 enriched B-cells were centrifuged on glass slides and stained with a EBV gp350-specific rat monoclonal antibody in 10% AB serum solution for 30 minutes. Antibody-labeled cells were stained by APAAP (alkaline phosphate anti-alkaline phosphate) method. Cytospin slides were analyzed by a computerized image analysis system (MDS, Applied Imaging) counting positively red stained cells.

### Quantification of cytokines

Cytokine levels in plasma samples were analyzed applying the Luminex system 200 (Luminex, TX, USA) together with the premixed 8-plex fluorokine X-Map kit (R&D Systems, MN, USA) comprising the cytokines IL-2, IL-4, IL-6, IL-8, IL-10, IL-17, IFN-yand TNF-α. Samples were collected at the indicated times points, stored at -20°C and measured in batch. The detection limit of the cytokines is 3.2 pg/ml.

### Detection of anti-EBNA-1 and VCA IgG antibodies

Antibodies in the patient's plasma specific for the EBV antigens EBNA-1 (Epstein-Barr Nuclear Antigen 1) and VCA (Viral-Capsid Antigen) were determined by IVD kit ELISA following the instructions of the manufacturer (medac GmbH, Germany). Briefly, plasma samples were incubated on pre-coated and pre-blocked microtiter plates. After a washing step bound EBV specific IgG antibodies were detected by anti-human IgG antibody conjugated to peroxidase. Finally, washed plates were developed using TMB (tetramethylbenzidine) substrate solution and the reaction was stopped with sulfuric acid. Microtiter plates were measured at 450 nm applying a Versamax plate reader (Molecular Devices, USA). EBV-specific antibody concentrations were calculated by interpolation on a standard curve. Results of > 11 AU/ml were considered as positive.

### Preparation of autologous cell vaccine

PBMCs and B-cell enrichment were performed as described above from 60 ml EDTA peripheral blood. All manipulations were performed under sterile conditions under a laminar air flow. 250 x 10³ cells of the B -cell fraction (in 0.5 ml PBS) and 500 x 10³ PBMCs (in 0.5 ml PBS) were filled in separate, sterile septum-sealed glass vials. Then, 1 µg of a trifunctional bispecific antibody (in 0.1ml PBS) with the specificities anti-CD20 x anti-CD3 was added to the B-cell fraction and the cells were incubated for 10 minutes at room temperature. Subsequently, the PBMC fraction was added to the pre-incubated autologous B cell fraction and incubated for another 10 minutes. Eventually, the entire cell preparation was applied subcutaneously back to the patient. For the boost application the same procedure was repeated.

The above embodiments can be combined arbitrarily, if appropriate. Further possible embodiments and implements of the invention comprise also combinations of features not explicitly mentioned in the foregoing or in the following with regard to the examples of the invention. Particularly, a person skilled in the art will also add individual aspects as improvements or additions to the respective basic form of the invention.

### Examples

The present invention will now be described in detail with reference to examples thereof. However, these examples are illustrative and do not limit the scope of the invention.

### Example 1. Trifunctional bispecific antibody mediated killing of enriched, autologous B-cells in vitro

Example 1 demonstrates the effective killing of enriched, autologous B-cells targeted by a CD20-specific trifunctional bispecific antibody *in vitro.* 250.000 enriched B-cells and 500.000 PBMC from a healthy donor were prepared as described in the method and materials section. The cells were mixed and incubated in the presence or absence of 1µg Bi20 which is a trifunctional bispecific anti-CD3 x anti-CD20 antibody (Figure 1). Cells were cultivated in a total volume of 1ml culture medium (RPMI 1640 medium supplemented with 8,9% FCS, 2mM L-Glutamine, 1mM Sodium-Pyruvate and 1 x Non-Essential-Amino-Acids) in 24 well plates at 37°C and 5% C0₂. After three days, cells were analyzed by flow cytometry using a FACS-Calibur and Cellquest pro software (Becton Dickinson, USA). B-cells were stained by PE (phycoerythrin)-conjugated anti-human CD20 monoclonal antibody (Caltag, USA) and quantified by histogram statistics. As shown in Figure 2A, efficiency of B-cell enrichment was over 80%. After three days, the detected B-cell population in the approaches without Bi20 was 9.24% in mean (n=2; Figure 2B). In contrast, only 3.75 % B-cells in mean (n=2) were quantified in the approaches where Bi20 was added (Figure 2C). In fact, a comparison of the histograms demonstrates that the distinct CD20+ B-cell population completely vanished. Thus, the addition of the trifunctional bispecific antibody Bi20 resulted in efficient and complete elimination of targeted B-cells by retargeted cellular cytotoxicity.

### Example 2 (chronic fatigue syndrome, chronic dry cough, night sweat)

A 49- year old woman with positive EBV-testing suffered from repeated fatigue episodes for ten years, lymphedema, chronic otitis media, night-sweat on and off. Moreover, the patient was diagnosed for a pancreatitis in May 2010 and developed a rectum carcinoma in July 2010. EBV was detected in tumor cells by PCR analysis and later in operation scar. The patient has chronic dry cough since 2000.

In an elaborated EBV diagnostic, e.g. a high frequency of EBER-1+2 CISH-positive B cells (>50%) could be assessed within an enriched B-cell fraction of the patient as well as slightly elevated IL-8 values.

Due to this diagnosis and unmet medical need, the patient decided to participate to a compassionate use treatment with an investigational therapeutic anti-EBV vaccine.

To prepare the vaccine cell preparation, 60 ml of EDTA peripheral blood was taken from the patient. First, lymphocytes were isolated by Ficoll gradient centrifugation. Subsequently, B cells were enriched from a fraction of the isolated peripheral blood mononuclear cells (PBMC) by immunomagnetic beads using a CD20 specific antibody.

After DNase digestion and several washing steps to remove the immunomagnetic beads an enriched B -cell fraction (>85%) could be produced.

250 x 10³ cells of the B -cell fraction (within 0.5ml sterile PBS) were then incubated with 1µg (0.1ml PBS) of Bi 20, a trifunctional bispecific antibody with the specificities anti-CD20 x anti-CD3 for 10 minutes.

Subsequently, 500 x 10³ PBMCs (within 0.5ml sterile PBS) were added to the pre-incubated autologous B cell fraction and incubated for another 10 minutes.

All manipulations were performed under sterile conditions under a laminar air flow at room temperature.

Eventually, the entire cell preparation was applied subcutaneously back to the patient (26.2.15).

For the boost application the same procedure was repeated (25.3.15 and 2.2.16).

The following diagnostic panel was assessed before, during and after treatment:

### Immunological Results:

**Cytokine measurement**

| Cytokines | before immunisat. 26.2.2015 | 24h after | 3 weeks | before boost | 48h after | 1 week | Reference |
|---|---|---|---|---|---|---|---|
| INF-gamma | 3.1 pg/ml | 3.1 | 3.1 | 3.1 | 3.1 | 3.1 | 3.1 |
| IL-10 | 3.1 | 3.1 | 3.1 | 3.1 | 3.1 | 3.1 | 3.1 |
| IL-17 | 3.1 | 3.1 | 3.1 | 3.1 | 3.1 | 3.1 | 3.1 |
| IL-2 | 2.9 | 2.9 | 2.9 | 2.9 | 2.9 | 2.9 | 2.9 |
| IL-4 | 4.3 | 4.3 | 4.3 | 4.3 | 4.3 | 4.3 | 4.3 |
| IL-6 | 5.6 | 5.6 | 5.6 | **13.1** | 5.6 | 5.6 | 5.6 |
| IL-8 | **7.8** | **6.0** | **10.0** | **10.4** | 3.9 | 3.9 | 3.9 |
| TNF-alpha | 5.4 | 5.4 | 5.4 | 5.4 | 5.4 | 5.4 | 5.4 |

| Cytokines | 01.06.2015 | 07.01.2016 | 21.01.2016 | 02.02.2016 | 16.03.2016 | Reference |
|---|---|---|---|---|---|---|
| INF-gamma | 3,1 | 3,1 | 3,1 | 3,1 | 3,1 | 3.1 |
| IL-10 | 3,1 | 3,1 | 3,1 | 3,1 | 3,1 | 3.1 |
| IL-17 | 3,1 | 3,1 | 3,1 | 3,1 | 3,1 | 3.1 |
| IL-2 | 2,9 | 2,9 | 2,9 | 2,9 | 2,9 | 2.9 |
| IL-4 | 4,3 | 4,3 | 4,3 | 4,3 | 4,3 | 4.3 |

| | | | | | | |
|---|---|---|---|---|---|---|
| IL-6 | 5,6 | 5,6 | 5,6 | 5,6 | 5,6 | 5.6 |
| IL-8 | **19,3** | 3,9 | 3,9 | 3,9 | 3,9 | 3.9 |
| TNF-alpha | 5,4 | 5,4 | 5,4 | 5,4 | 5,4 | 5.4 |

**Humoral anti-EBV response**

| | | Before immunization (26.2.15) | Before boost (25.3.15) | Follow up (1.4.15) | Follow up (1.6.15) |
|---|---|---|---|---|---|
| Anti-EBNA-1 ELISA | AU/ml | 125.4 | 130.3 | 116.7 | **159.4** |
| Anti-VCA-ELISA | AU/ml | 36.6 | 35.7 | 34.5 | 36.5 |

**PCR analysis for EBV**

| Date | 25.11.2013 | 30.4.2014 | 18.12.2014 | 29.4.2015 |
|---|---|---|---|---|
| EBV specific PCR * | positive | positive | positive | **negative** |

| | | | | |
|---|---|---|---|---|
| *analysis was performed with patient's PBMCs isolated by Ficoll gradient | | | | |

### Clinical Results:

After the second application of the cell preparation (boost), the cough was improved and EBV was tested in operation scar and in blood as negative (PCR analysis). Moreover, the patient described an improvement of night sweats and felt to have more energy as well as improvement in the ability to concentrate. Furthermore, since the last treatment, no severe infections and a normalization of bowl movements were observed. EBV infected B cells in the patient are strikingly decreased (Figure 3).

### Example 3 (Insulin resistance, Diabetes type 2, potency problems)

A 50-year old man positively tested for EBV and Herpes virus type-1,- 2, CMV and chlamydia pneumonia suffered from metabolic syndrome with high insulin resistance, elevated liver enzymes, metabolic dysfunction of liver, hyper cholesterinemia, high triglycerides, sleeping disturbances, fatigue, lack of concentration and potency problems.

In an elaborated EBV diagnostic, e.g. a high frequency of EBNA-1+2 CISH-positive B cells (50-75%) could be assessed within an enriched B-cell fraction of the patient. Due to this diagnosis and unmet medical need, the patient decided to participate to a compassionate use treatment with an investigational therapeutic anti-EBV vaccine, as also disclosed in Example 2.

A cell preparation similar to Examples 1 and 2 was produced *ex vivo* and applied subcutaneously back to the patient (13.5.15).

For the boost application the same procedure was repeated (10.6.15).

| | HOMA (<2) |
|---|---|
| 24.2.15 | 6.6 |
| 13.5.15 | 6.0 |
| 29.7.15 | 4.5 |

HOMA: Homeostasis Model Assessment, HOMA-Index = Insulin (sober, µU/ml) x blood sugar sober, mg/dl) / 405, HOMA-Index>2 indicator for insulin resistance.

### Clinical Results:

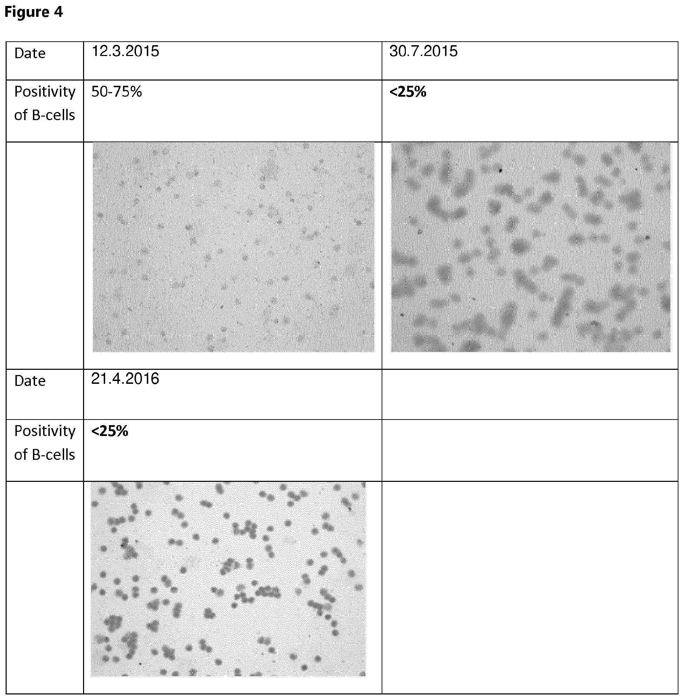

The patient showed improved symptoms of fatigue and potency, liver enzymes, triglycerides as well as improved cholesterol values and insulin resistance. The patient could sleep better. EBV infected B cells in the patient are strikingly decreased (Figure 4).

### Example 4 (skin inflammation, sleeping disorders, repetitive infections)

A 41-year old woman) positively tested for EBV (PCR) suffered from chronic bronchitis, metabolic syndrome, vegetative dystonia, extreme fatigue, obstipation and lots of gas, lactose intolerance, weight loss, sleeping disturbance, chronic flue, massive acne-like skin problems at face and back, night sweats, swollen lymph glands submandibular and cervical. Moreover, positively tested with PCR for Herpes -1. High IgG titers for CMV, herpes- 6 and herpes-1 and -2.

In an elaborated EBV diagnostic, e.g. a high frequency of EBNA-1+2 CISH-positive B cells (>75%) could be assessed within an enriched B-cell fraction of the patient as well as slightly elevated IL-8 values (see table).

Due to this diagnosis and unmet medical need, the patient decided to participate to a compassionate use treatment with an investigational therapeutic anti-EBV vaccine, as also disclosed in Example 2.

A cell preparation similar to Examples 1 and 2 was produced *ex vivo* and applied subcutaneously back to the patient (26.3.15).

For the boost application the same procedure was repeated (5.5.15).

The following diagnostic panel was assessed before, during and after treatment:

### Immunological Results:

**Cytokine measurement**

| Cytokines | before immunisat. (26.3.2015) | 24h after | before boost (5.5.15) | 24h after | Follow up (16.6.15) | | Reference |
|---|---|---|---|---|---|---|---|
| INF-gamma | 3.1 pg/ml | 3.1 | 3.1 | 3.1 | 3.1 | | 3.1 |
| IL-10 | 3.1 | 3.1 | 3.1 | 3.1 | 3.1 | | 3.1 |
| IL-17 | 3.1 | 3.1 | 3.1 | 1.1 | 3.1 | | 3.1 |
| IL-2 | 2.9 | 2.9 | 2.9 | 2.9 | 2.9 | | 2.9 |
| IL-4 | 4.3 | 4.3 | 4.3 | 4.3 | 4.3 | | 4.3 |
| IL-6 | 5.6 | 5.6 | 5.6 | 5.6 | 5.6 | | 5.6 |
| IL-8 | **10.9** | **9.4** | 3.9 | 3.9 | **16.5** | | 3.9 |
| TNF-alpha | 5.4 | 5.4 | 5.4 | 5.4 | 5.4 | | 5.4 |

**Humoral anti-EBV response**

| | | Before immunization (26.3.15) | 24h after | Before boost (5.5.15) | Follow up (16.6.15) |
|---|---|---|---|---|---|
| Anti-EBNA-1 ELISA | AU/ml | 12.6 | 8.7 | 10.9 | 10.8 |
| Anti-VCA-ELISA | AU/ml | 95.7 | 88.3 | **121.8** | **120.9** |

### Hematological Results:

**PCR analysis for EBV**

| Date | 14.1.15 | 31.8.15 | |
|---|---|---|---|
| EBV specific PCR * | Positive (30.58) | 33.71 | |

| | | | |
|---|---|---|---|
| *analysis was performed with patient's PBMCs isolated by Ficoll gradient | | | |

**Staining of enriched B-cells (CD20, 2.5 x 10e5) with anti-gp350 antibody APAAP technique**

| | Before Immunization (21.1.15) | After immunization and boost (25.8.15) |
|---|---|---|
| gp350 pos. cells | 10 | **0** |

### Clinical Results:

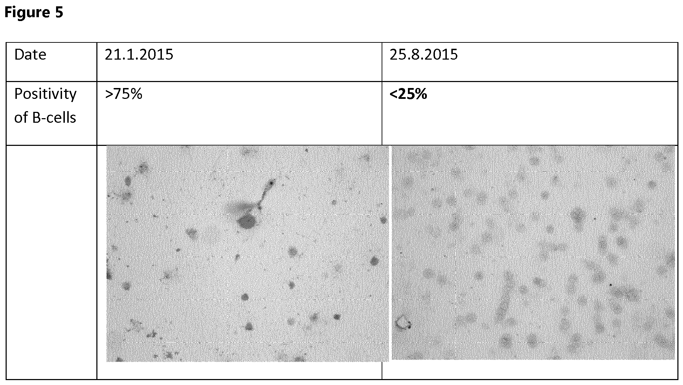

Improvement of symptoms of skin inflammation and fatigue were observed. The rate of bronchial infections significantly decreased. The patient could sleep normally again without night sweats. Weight was improved as well as vegetative dystonia (nervous stomach was improved) with normalization of bowl movements. EBV infected B cells in the patient are strikingly decreased (Figure 5).

### Example 5 (polyneuropatic pain, edemas in fingers, toes and the face, repeated infections, sleeping disorders, swollen lymphglands, endometriosis, chronic cystitis, chronic inflammation of milk ducts)

A-48 year old woman positively tested for EBV suffered from Endometriosis, fatigue, memory and concentration problems, sleeping problems, night sweats, swollen lymphglands inguinal and submandibular, chronic cystitis, chronic inflammation of milk ducts in both breasts, polyneuropatic pain in fingers and toes, lymphedema in legs, fingers and face and allergies (running nose). The patient developed a bladder carcinoma in 2014 (stage 1). The patient showed high IGG for herpes type-6, CMV and chlamydia pneumonia.

In an elaborated EBV diagnostic, e.g. a high frequency of EBER-1+2 CISH-positive B cells (>75%) could be assessed within an enriched B -cell fraction of the patient as well as slightly elevated IL-8 values (see table).

Due to this diagnosis and unmet medical need, the patient decided to participate to a compassionate use treatment with an investigational therapeutic anti-EBV vaccine, as disclosed in Example 2.

A cell preparation similar to Examples 1 and 2 was produced *ex vivo* and applied subcutaneously back to the patient (24.3.15).

For the boost application the same procedure was repeated (5.5.15).

The following diagnostic panel was assessed before, during and after treatment:

### Immunological Results:

**Cytokine measurement**

| Cytokines | | before immunisat. (24.3.15) | 24h after | before boost (5.5.15) | 6.5.15 | 24h after | Follow up (29.5.15) | 24.8.15 | Reference |
|---|---|---|---|---|---|---|---|---|---|
| INF-gamma | | 3.1 pg/ml | 3.1 | 3.1 | | 3.1 | 3.1 | | 3.1 |
| IL-10 | | 3.1 | 3.1 | 3.1 | | 3.1 | 3.1 | | 3.1 |
| IL-17 | | 3.1 | 3.1 | 3.1 | | 3.1 | 3.1 | | 3.1 |
| IL-2 | | 2.9 | 2.9 | 2.9 | | 2.9 | 2.9 | | 2.9 |
| IL-4 | | 4.3 | 4.3 | 4.3 | | 4.3 | 4.3 | | 4.3 |
| IL-6 | | 5.6 | 5.6 | 5.6 | | 5.6 | 5.6 | | 5.6 |
| IL-8 | | **10.4** | **9.4** | 3.9 | 140 (<62 normal) | 3.9 | **34.4** | 55 (<62 normal | 3.9 |
| TNF-alpha | | 5.4 | 5.4 | 5.4 | | 5.4 | 5.4 | | 5.4 |

**Humoral anti-EBV response**

| | | Before immunization (24.3.15) | Before boost (5.5.15) | Follow up (29.5.15) | Follow up (24.8.15) |
|---|---|---|---|---|---|
| Anti-EBNA-1 ELISA | AU/ml | 58.1 | **75.1** | **95** | **88.2** |
| Anti-VCA-ELISA | AU/ml | 179.9 | **197.1** | **232.9** | **276.8** |

**Staining of enriched B-cells (CD20, 2.5 x 10e5) with anti-gp350 antibody APAAP technique**

| | Before Immunization (20.1.15) | After immunization and boost (25.8.15) | Follow up (7.4.16) |
|---|---|---|---|
| gp350 pos. cells | 3 | **0** | **0** |

### Clinical Results:

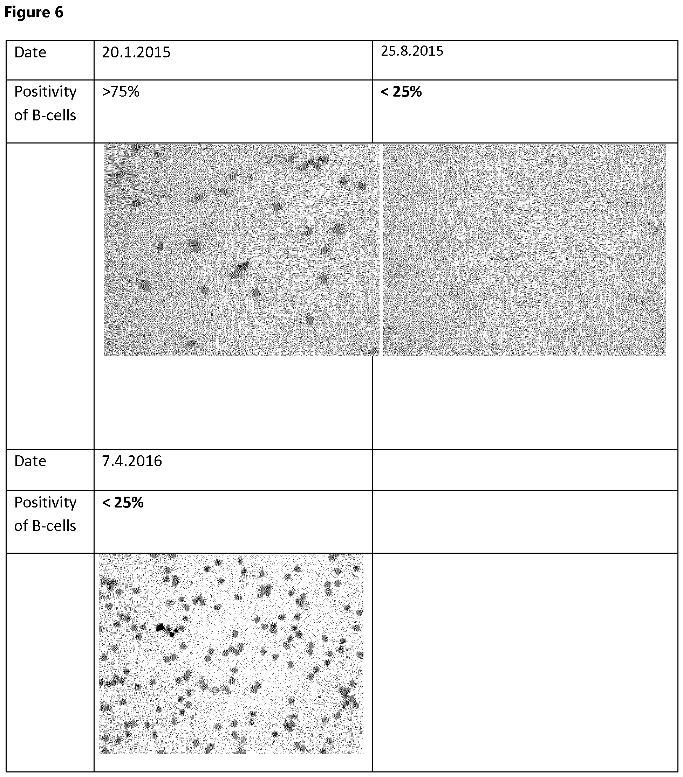

After the boost, the patient showed improvement in concentration and memory and normalization of sleep. The chronic inflammation of milk ducts in breast dissolved as well as fatigue symptoms. The polyneuropatic pain was improved as well as edemas in fingers, toes and the face. An improvement was also observed of allergic symptoms (running nose) and the infection rate decreased. EBV infected B cells in the patient are strikingly decreased (Figure 6).

### Example 6 (Insulin resistance, Diabetes type2, Sjögren- syndrome, rheumatoid arthritis, Hashimoto, hair loss, memory problems, dry eyes and mouth)

A 58-year old woman positively tested for EBV suffered from Sjögren- syndrome, rheumatoid arthritis, Hashimoto, hair loss, memory problems, dry eyes and mouth, insulin resistance.

In an elaborated EBV diagnostic, e.g. a high frequency of EBER-1+2 CISH-positive B cells (50-75%) could be assessed within an enriched B-cell fraction of the patient.

Due to this diagnosis and unmet medical need, the patient decided to participate to a compassionate use treatment with an investigational therapeutic anti-EBV vaccine, as also disclosed in Example 2.

A cell preparation similar to Examples 1 and 2 was produced *ex vivo* and applied subcutaneously back to the patient (29.7.15).

For the boost application the same procedure was repeated (15.9.15).

The following diagnostic panel was assessed before, during and after treatment:

### Immunological Results:

**Cytokine measurement**

| Cytokines | 19.5.15 | before immunisat. (29.7.15) | 24h after | before boost (15.9.15) | 24h after | 26.10.15 | Reference |
|---|---|---|---|---|---|---|---|
| INF-gamma | | 3.1 pg/ml | 3.1 | 3.1 | 3.1 | | 3.1 |
| IL-10 | | 3.1 | 3.1 | 3.1 | 3.1 | | 3.1 |
| IL-17 | | 3.1 | 3.1 | 3.1 | 3.1 | | 3.1 |
| IL-2 | | 2.9 | 2.9 | 2.9 | 2.9 | | 2.9 |
| IL-4 | | 4.3 | 4.3 | 4.3 | 4.3 | | 4.3 |
| IL-6 | | 5.6 | 5.6 | 5.6 | 5.6 | | 5.6 |
| IL-8 | 154pg/ml | 3.9 | 3.9 | 3.9 | 3.9 | 45.6 (<62 normal) | 3.9 |
| TNF-alpha | | 5.4 | 5.4 | 5.4 | 5.4 | | 5.4 |

**Humoral anti-EBV response**

| | | Before immunization (29.7.15) | Before boost (15.9.15) | Follow up (26.10.15) |
|---|---|---|---|---|
| Anti-EBNA-1 ELISA | AU/ml | 72.3 | 126.4 | **148.2** |
| Anti-VCA-ELISA | AU/ml | 22.5 | 22.5 | 22.1 |

**Staining of enriched B-cells (CD20, 2.5 x 10e5) with anti-gp350 antibody APAAP technique**

| | | Before Immunization (29.6.15) | | After immunization and boost (26.10.15) | |
|---|---|---|---|---|---|
| gp350 pos. cells | | **12** | | 0 | |
| | 12.5.15 | 19.5.15 | 29.7.15 | 27.10.15 | |
| HOMA (<2) | **3.1** | | **2.1** | 1 | |
| CRP (highly sensitive) | | 20.1 | | | |
| IL-1 -R | | 15% over control | | | |
| TNF alpha -R | | 15% over | | | |
| IL-15-R | | 15% over | | | |
| IL-12-R | | 10% over | | | |

| | | | | | |
|---|---|---|---|---|---|
| HOMA: Homeostasis Model Assessment, HOMA-Index = Insulin (sober, µU/ml) x blood sugar sober, mg/dl) / 405, HOMA-Index>2 indicator for insulin resistance. | | | | | |

### Clinical Results:

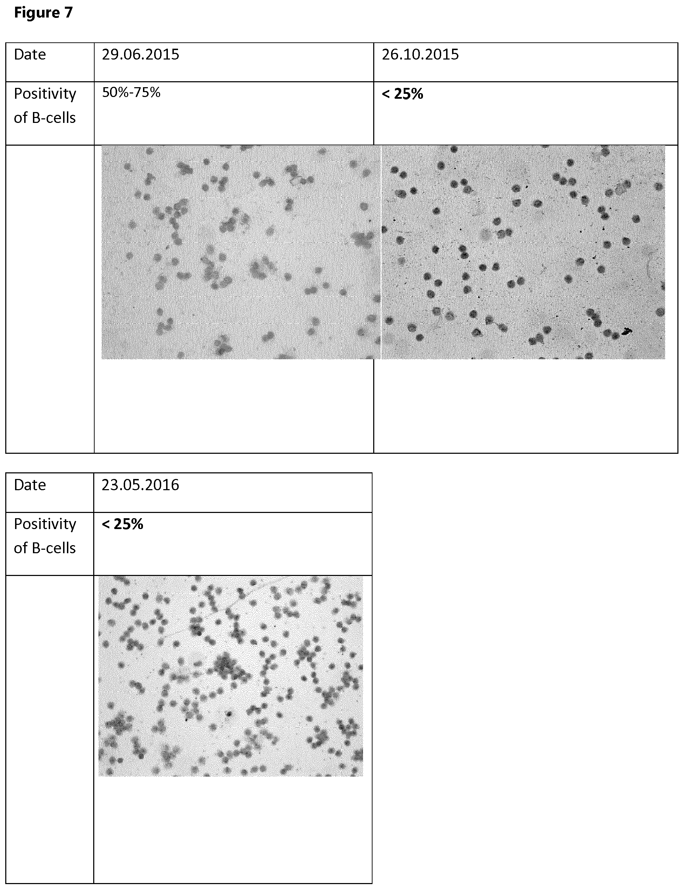

Significant decrease of insulin resistance and infection rate was observed. Skin discoloration which is a typical Sjögren-syndrome, was improved. The hair loss was stopped. EBV infected B cells in the patient are strikingly decreased (Figure 7).

### Example 7 (Rheumatoid Arthritis, insulin resistance)

A 44-year old man positively tested for EBV suffered from rheumatoid polyarthritis, metabolic syndrome, and insulin resistance.

In an elaborated EBV diagnostic, e.g. a medium frequency of EBER-1+2 CISH-positive B cells (25-50%) could be assessed within an enriched B-cell fraction of the patient. In addition, an exceptionally high number (38) of gp350 positive B-cells was detected speaking for an active EBV reactivation in that patient.

Due to this diagnosis and unmet medical need, the patient decided to participate to a compassionate use treatment with an investigational therapeutic anti-EBV vaccine, as also disclosed in Example 2.

A cell preparation similar to Examples 1 and 2 was produced *ex vivo* and applied subcutaneously back to the patient (18.8.15).

For the boost application the same procedure was repeated (15.9.15).

The following diagnostic panel was assessed before, during and after treatment:

### Immunological Results:

**Humoral anti-EBV response**

| | | Before immunization (18.8.15) | Before boost (15.9.15) | Follow up (03.11.15) |
|---|---|---|---|---|
| Anti-EBNA-1 ELISA | AU/ml | 314 | **357** | **287.5** |
| Anti-VCA-ELISA | AU/ml | 371.5 | 389 | **385** |

**Staining of enriched B-cells (CD20, 2.5 x 10e5) with anti-gp350 antibody APAAP technique**

| | Before immunization (30.6.15) | Six weeks after immunization and boost (2.11.15) | Follow up 11.5.16 | Follow up 7.9.16 | Follow up 22.5.17 | Follow up 30.11.17 |
|---|---|---|---|---|---|---|
| gp350 pos. cells | **38** | 0 | 1 | 2 | 0 | 0 |

| | 29.6.15 | 2.11.15 | 9/2016 | | |
|---|---|---|---|---|---|
| HOMA (<2) | **3.6** | 1.1 | | | |
| RF quantitative (IgM <18) | **244.6** | **200.4** | **300** | | |
| ANA-IFT (1:80) | **1:640** | **1:160** | **1:160** | | |
| Interleukin-5 (<1pg/ml) | **5.2** | 0.1 | 0.1 | | |
| Interleukin-22 (<1pg/ml) | **6** | 0.1 | 0.1 | | |
| Interferon-gamma (<1pg/ml) | **20.2** | 0.1 | 0.1 | | |

| | | | | | |
|---|---|---|---|---|---|
| HOMA: Homeostasis Model Assessment, HOMA-Index = Insulin (sober, µU/ml) x blood sugar sober, mg/dl) / 405, HOMA-Index>2 indicator for insulin resistance. RF, rheuma factor: autoantibodies of subclass IgM, binding to constant Fc-regions of antibodies. ANA-IFT: anti -nucleus antibodies, immunofluorescence -detection, indicator for autoimmune diseases like e.g. RA. | | | | | |

### Clinical Results:

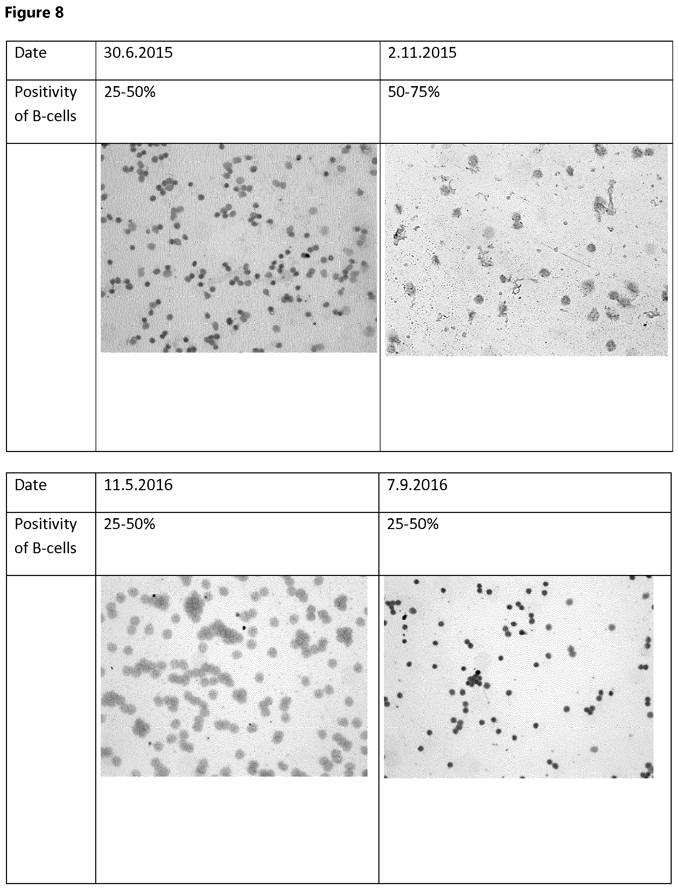

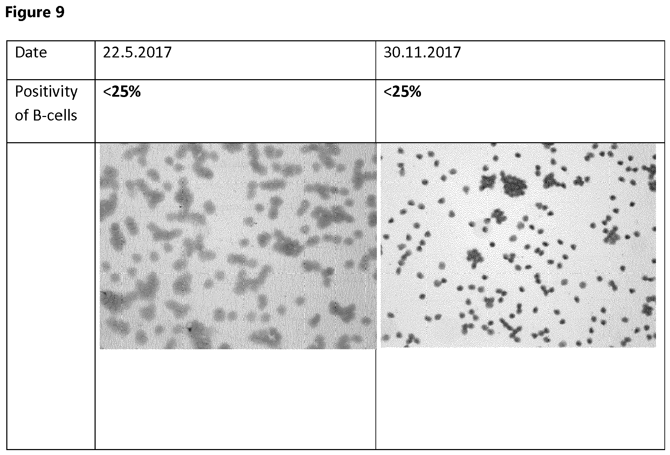

Seven weeks after the boost immunization (15.9.2015), the rheumatoid arthritis symptoms were significantly improved which is consistent with a reduction of inflammatory cytokines IL-5, IL-22 and INF-gamma and rheuma-associated laboratory values (RF, ANA-IFT). No analgetics necessary since September 2015 (for at least six months follow up). Swellings of hand joints significantly reduced. Insulin resistance was normalized. During 2016, no relapse of the rheumatoid arthritis was observed and Hashimoto syndrome remained stable as well as the eczema (rosacea). EBV infected B cells in the patient are strikingly decreased with the ongoing of the therapy (Figures 8 and 9).

### Example 8 (Diabetes type 1)

A 9-year old girl positively tested for EBV suffered from diabetes type 1 insulin resistance.

In an elaborated EBV diagnostic, e.g. a high frequency of EBNA-1+2 CISH-positive B cells (25-50%) could be assessed within an enriched B -cell fraction of the patient.

Due to this diagnosis and unmet medical need, the patient decided to participate to a compassionate use treatment with an investigational therapeutic anti-EBV vaccine, as also disclosed in Example 2.

A cell preparation similar to Examples 1 and 2 was produced *ex vivo* and applied subcutaneously back to the patient (11.11.15).

**Staining of enriched B-cells (CD20, 2.5 x 10e5) with anti-gp350 antibody APAAP technique**

| | 9.11.15 | After immunization 21.1.16 | Follow up 27.6.16 |
|---|---|---|---|
| gp350 pos. cells | **1** | 0 | 0 |

### Clinical Results:

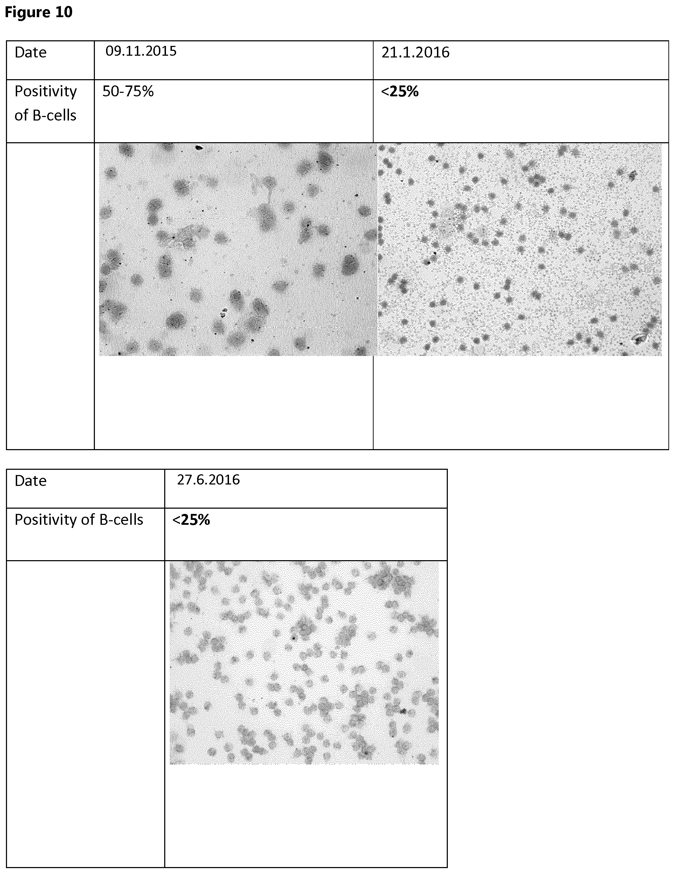

After the treatment the growth was improved as well as general energy and sleeping quality. The HBAlc value remained stable and no severe infections were observed during follow up. EBV infected B cells in the patient are strikingly decreased (Figure 10).

### Example 9 (vaginal lichen)

A 65-year old woman positively tested for EBV suffered from vaginal lichen, chronic vaginal infections, chronic vaginal pain, chronic fatigue and bad concentration.

In an elaborated EBV diagnostic, e.g. a high frequency of EBER-1+2 CISH-positive B cells (50-75%) could be assessed within an enriched B -cell fraction of the patient.

Due to this diagnosis and unmet medical need, the patient decided to participate to a compassionate use treatment with an investigational therapeutic anti-EBV vaccine, as also disclosed in Example 2.

A cell preparation similar to Examples 1 and 2 was produced *ex vivo* and applied subcutaneously back to the patient at 22.10.15.

For the boost application the same procedure was repeated (19.11.15).

The following diagnostic panel was assessed before, during and after treatment:

| | 10/2015 | 01/2016 | 05/2016 | 10/2016 | |
|---|---|---|---|---|---|
| HOMA (<1) | - | - | - | - | |
| RF quantitative (IgM <18) | - | - | - | - | |
| ANA-IFT (1:80) | **1:80** | <1:80 | <1:80 | 1:80 | |
| Interleukin-5 (<1pg/ml) | **3.8** | 2.3 | 0.1 | 0.1 | |
| Interleukin-22 (<1pg/ml) | 0.1 | 0.1 | 0.1 | 0.1 | |
| Interferon-gamma (<1pg/ml) | **69.4** | **59.8** | **23.2** | 0.1 | |

| | | | | | |
|---|---|---|---|---|---|
| HOMA: Homeostasis Model Assessment, HOMA-Index = Insulin (sober, µU/ml) x blood sugar sober, mg/dl) / 405, HOMA-Index>2 indicator for insulin resistance. RF, rheuma factor: autoantibodies of subclass IgM, binding to constant Fc-regions of antibodies. ANA-IFT: anti -nucleus antibodies, immunofluorescence -detection, indicator for autoimmune diseases like e.g. RA. | | | | | |

### Clinical Results:

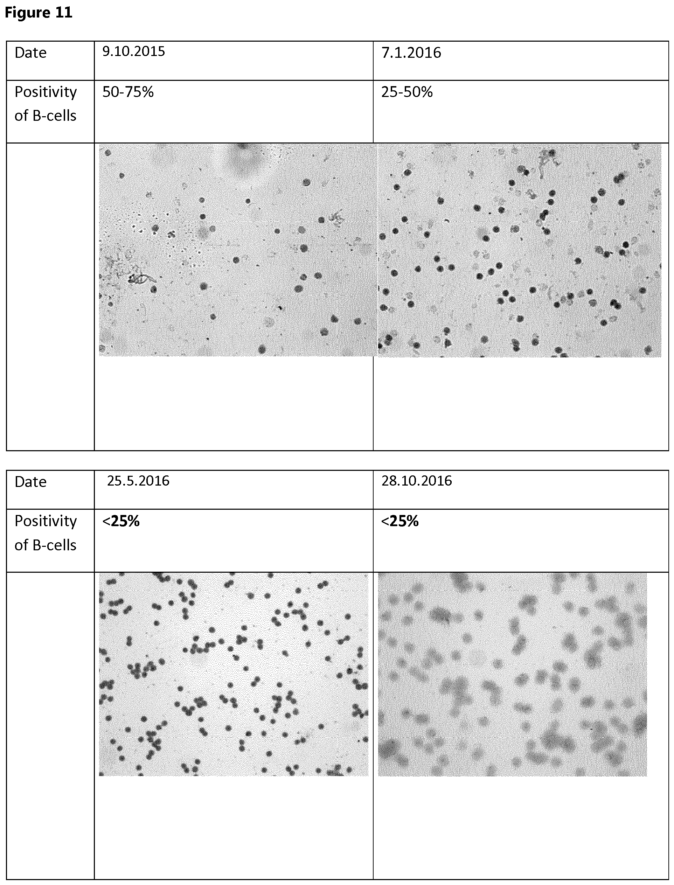

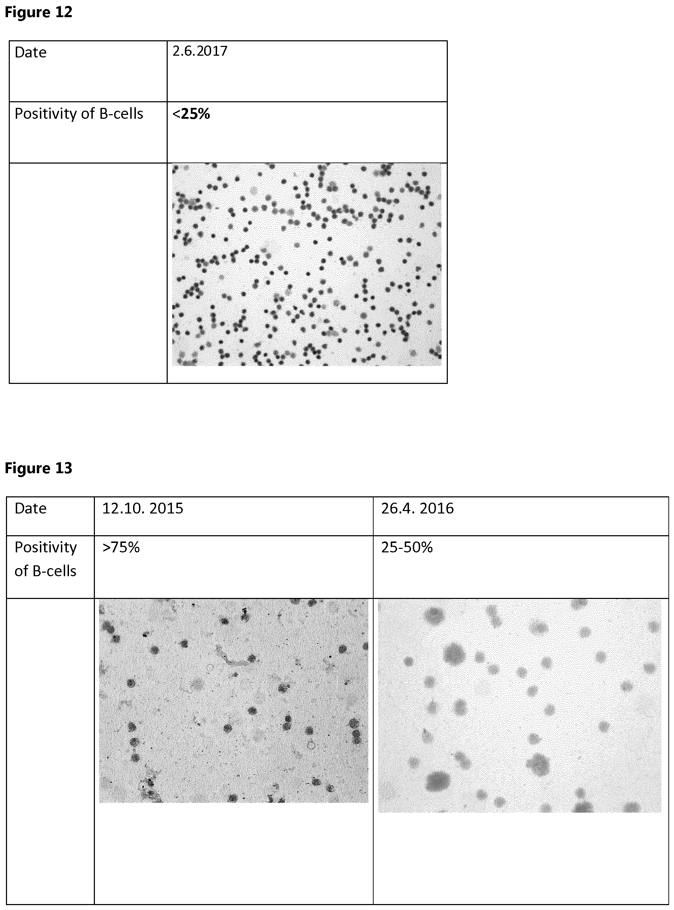

In October 2015 the patient presented with chronic vaginal infections, chronic vaginal pain, chronic fatigue and bad concentration. During post treatment examinations at 01/2016, 05/2016 and 10/2016 the situation of the patient constantly improved which was also confirmed by laboratory parameters as a reduction of the B-cell EBV load as well as cytokine titers was observed. At the last examination in June/2017 the B-cell EBV load was still <25% and the patient had again normal energy, no infections and a reduction of vaginal lichen and no vaginal pain. EBV infected B cells in the patient are strikingly decreased with the ongoing of the therapy (Figures 11 and 12).

### Example 10 (chronic cystitis and prostatitis)

A -65 year old man positively tested for EBV suffered from chronic cystitis and prostatitis, depressions, fatigue, coughs and insulin resistance.

In an elaborated EBV diagnostic, e.g. a high frequency of EBER-1+2 CISH-positive B cells (>75%) could be assessed within an enriched B -cell fraction of the patient.

Due to this diagnosis and unmet medical need, the patient decided to participate to a compassionate use treatment with an investigational therapeutic anti-EBV vaccine, as also disclosed in Example 2.

A cell preparation similar to Examples 1 and 2 was produced *ex vivo* and applied subcutaneously back to the patient at 16.10.15.

For the boost application the same procedure was repeated (27.4.17).

The following diagnostic panel was assessed before, during and after treatment:

**Staining of enriched B-cells (CD20, 2.5 x 10e5) with anti-gp350 antibody APAAP technique**

| | Before immunization (12.10.15) | After immunization (26.4.16) | Follow Up 24.4.17 | |
|---|---|---|---|---|
| gp350 pos. cells | **2** | 0 | 4 | |

### Clinical Results:

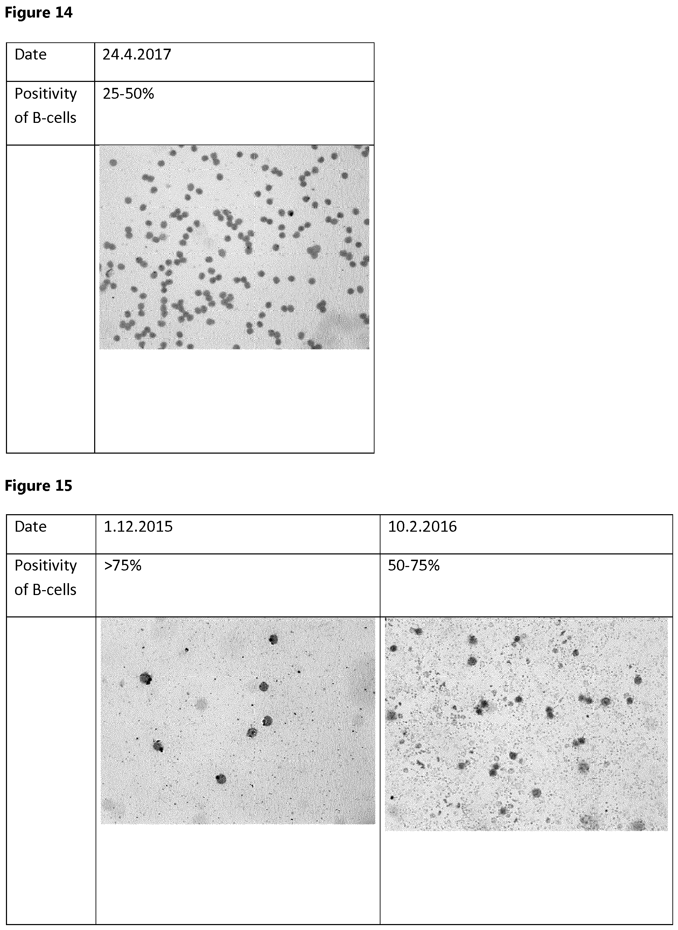

After treatment during a visit in May 2016, an improvement in concentration and focusing was observed. The prostatitis stopped with one re-appearance in March 2016 which could be treated with antibiotics. The cystitis was improved, as well as the general infection rate. Whereas the anti-depression medication could be reduced from 3 different anti-depressiva to one anti-depressiva, the barbiturates could also be reduced. A reduction of the fibromyalgic pain was also observed. EBV infected B cells in the patient are strikingly decreased with the ongoing of the therapy (Figures 13 and 14).

### Example 11 (Multiple Sclerosis)

A 40-year old woman positively tested for EBV suffered from Multiple Sclerosis (MS). In an elaborated EBV diagnostic, e.g. a high frequency of EBER-1+2 CISH-positive B cells (>75%) could be assessed within an enriched B-cell fraction of the patient.

Due to this diagnosis and unmet medical need, the patient decided to participate to a compassionate use treatment with an investigational therapeutic anti-EBV vaccine, as also disclosed in Example 2.

A cell preparation similar to Examples 1 and 2 was produced *ex vivo* and applied subcutaneously back to the patient (3.12.15).

For the boost application the same procedure was repeated (6.11.16).

The following diagnostic panel was assessed before, during and after treatment:

**Staining of enriched B-cells (CD20, 2.5 x 10e5) with anti-gp350 antibody APAAP technique**

| | Before immunization (1.12.15) | Nine weeks after immunization (10.2.16) | Follow up 30.5.16 | Follow up 2.10.17 | Follow up 16.4.18 |
|---|---|---|---|---|---|
| gp350 pos. cells | **0** | 0 | 3 | 1 | 0 |

### Clinical Results

The patient had a progressive multiple sclerosis first diagnosed middle of 2011 through MRI and lumbar aspiration, with symptoms of heavy legs, numb feet, difficulties with walking and balancing, and also down shooting pain in both legs. The patient got therapy with Avonex 30 mg, cortisone. At the beginning of 2012, the patient developed a progression, with more active MS lesions C4/5 (cervical spine) seen in MRI. Therapy subsequently changed to interferon. The patient developed symptoms of concentration problems, sleep disturbance, fatigue, increasing numbness mainly right leg and foot, more dysbalanced in walking. In December 2013, under that further progression and development of depression, multiple eczema (first time diagnosed), with more progressive MS lesions C4/5 seen in MRI. Therapy changed to refib, cortisone. The patient had symptoms of only walking less than 1 km through increased pain, and must walking with help, weakness right arm and concentration problems. In March 2014, a progress of the disease was seen in MRI with a new lesion cerbellum-area (brain) and thoracic spine (T2 = exact localization: second thoracic vertebra) as well as new progression of the lesion C4/5. No standard therapy was available any more. Trial with fampridine, 2x dendritic cell therapy.

At the beginning of the trial therapy, the patient had symptoms of dizziness, increase of fatigue, heaviness in legs, more numbness in the right side, and sleeping disturbance.

In July 2014, a progression of the disease was seen in MRI of the C4/5 lesion, with increased numbness, neurodegeneration and demyelination process in the brain. Therapy with photopheresis and anti-inflammatory infusion was started. The patient had symptoms of losing control of right leg, increased fatigue, increased memory and concentration problems, more severe sleeping problems.

In February 2015: stable disease C4/5 was seen in MRI, T2 is less active, cerebellum is normal, with less eczema. Ongoing anti-inflammatory therapy with herbal extracts (curcuma) and omega3 - fatty acids. The patient showed symptoms of improved walking, less numbness, better sleep, and more energy.

In November 2015: Progression was seen in MRI in C4/5, progression T2. The patient had symptoms of more fatigue, increased numbness in the right foot, less sensitivity of right arm, tingling in fingers right hand, Vertigo, and dizziness. First therapeutic EBV-vaccination (compassionate use) was started in early December 2015.

In June 2016: in MRI stable disease C4/5 and T2 were seen. The patient showed symptoms of further improvement of numbness, more balanced, no vertigo, no dizziness, more energy.

In October 2016: C4/5 stable disease, T2 mild progression was seen in MRI. The patient showed symptoms of increased numbness in feet and legs, no eczema, energy good, better sleeping.

In November 06, 2016: second therapeutic EBV- vaccination was started.

In March 2017: C4/5 in MRI stable. The patient had symptoms of less numbness.

In September 2017: in MRI, C4/5 inactive lesion was seen, T2 : lesion was not seen.

In April 2018: MRI: the patient showed stable appearance.

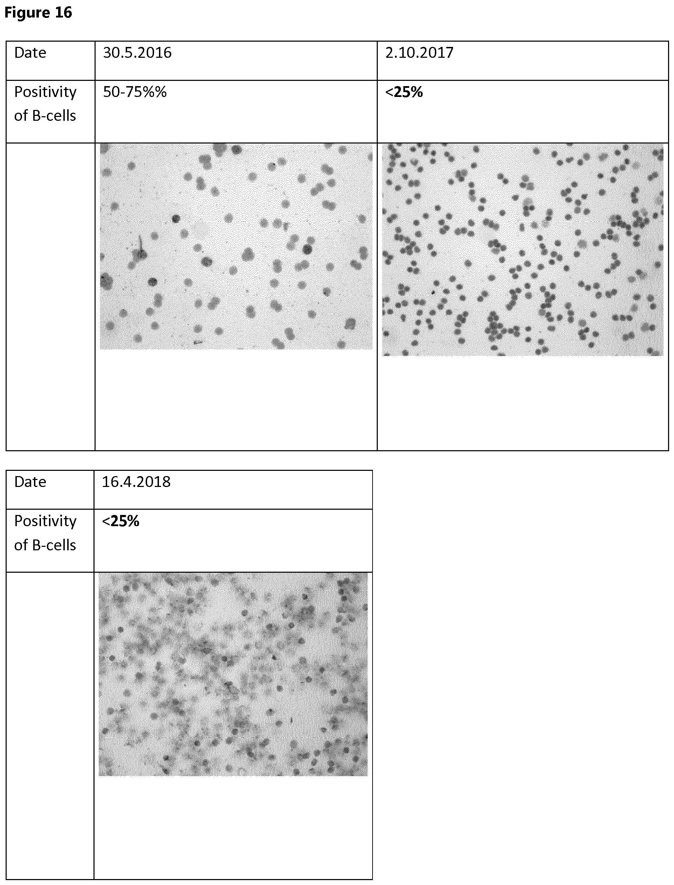

EBV infected B cells in the patient are strikingly decreased with the ongoing of the EBV-vaccination therapy (Figures 15 and 16).

## Claims

1. An isolated trifunctional bispecific antibody for use in a method of treatment of a patient suffering from a reactivation of Epstein-Barr virus (EBV) in B cells, **characterized in that** the antibody has the following properties:
(a) binding to a B cell via a B cell surface antigen;
(b) binding to a T cell via a T cell surface antigen;
(c) binding via its Fc-portion to an Fc receptor-positive cell.

2. The antibody for use according to claim 1, wherein the antibody is a rat/mouse bispecific antibody.

3. The antibody for use according to claim 1 or 2, wherein the antibody comprises a binding site in its Fc-portion for Fcγ receptor type I, IIa and/or III.

4. The antibody for use according to any one of claims 1 to 3, wherein the antibody is capable of binding monocytes, macrophages, dendritic cells, natural killer cells and/or activated neutrophils by their Fcγ receptor type I, IIa and/or III.

5. The antibody for use according to any one of claims 1 to 4, wherein the antibody is selected from at least one member of the following group of isotype combinations in its Fc-portion:
rat-IgG2b/mouse-IgG2a;
rat-IgG2b/mouse-IgG2b;
rat-IgG2b/human-IgG1;
human-IgG1/human-IgG1-[hinge]-human-IgG3*--[CH2-CH3], wherein *= caucasian allotypes G3m(b+g) = no binding to protein A.

6. The antibody for use according to any one of claims 1 to 5, wherein the B cell surface antigen is selected from the group consisting of CD 19, CD20, CD21, CD22, CD23, CD24, CD37, CD38, CD72, CD75, CD78, CD79 and CD80, preferably the B cell surface antigen is CD20.

7. The antibody for use according to any one of claims 1 to 5, wherein the T cell surface antigen is selected from a group consisting of CD2, CD3, CD4, CD8, CD28, CD40L and CD44, preferably the T cell surface antigen is CD3.

8. The antibody for use according to any one of claims 1 to 5, wherein the antibody is selected from a group consisting of anti-CD3 x anti-CD20, anti-CD3 x anti-CD19, anti-CD3 x anti-CD22, anti-CD3 x anti-GD38 bispecific antibodies, preferably the antibody is anti-CD3 x anti-CD20 bispecific antibody, and further preferably with the isotype combination rat-IgG2b/mouse-IgG2a.

9. The antibody for use according to any one of claims 1 to 8, wherein the antibody is administered in an amount of 0.1 - 100 µg.

10. A pharmaceutical composition comprising the antibody and optionally pharmaceutically acceptable carriers and/or excipients for use in a method of treatment of patients suffering from a reactivation of EBV in B cells according to any one of claims 1 to 9.

11. The pharmaceutical composition for use according to claim 10, the method further comprising an autologous cell preparation of enriched B cells of a patient suffering from a reactivation of EBV in B cells, and optionally additionally comprising an autologous cell preparation of mononuclear cells of peripheral blood (PBMC) of the same patient, wherein further optionally the pharmaceutical composition is in the form of "kit-of-parts".

12. The pharmaceutical composition for use according to claim 11, wherein said B cells are incubated with said trifunctional bispecific antibody for a time-period sufficient to establish a physical interaction between said trifunctional bispecific antibody and B cells.

13. An ex-vivo method for preparing the pharmaceutical composition for use in the method of treatment of reactivation of EBV in B cells according to claims 10 to 12, comprising an incubation step comprising contacting autologous B cells, from an autologous cell preparation of enriched B cells, of a patient suffering from a reactivation of EBV in B cells with said trifunctional bispecific antibody for a time-period sufficient to establish a physical interaction between said trifunctional bispecific antibody and B cells.

14. The ex-vivo method for preparing a pharmaceutical composition for use according to claim 13, further comprising
an incubation step comprising adding PBMC of the same patient with reactivation of EBV in B cells to the incubated autologous B cells according to claim 13, for a time-period sufficient to establish a physical interaction between said trifunctional bispecific antibody and PBMC.

15. The ex-vivo method for preparing a pharmaceutical composition for use according to claim 13 or 14, wherein the time period for incubation is between 1 min to 60 min.
